(19) Europäisches Patentamt
European Patent Office
Office européen des brevets

(11) EP 4 037 654 B1

(12) **EUROPEAN PATENT SPECIFICATION**

(45) Date of publication and mention of the grant of the patent:
**30.10.2024 Bulletin 2024/44**

(21) Application number: **20786275.6**

(22) Date of filing: **01.10.2020**

(51) International Patent Classification (IPC):
***A61K 8/85*** (2006.01) ***A61Q 17/04*** (2006.01)
***A61K 8/49*** (2006.01)

(52) Cooperative Patent Classification (CPC):
**A61K 8/85; A61K 8/4966; A61Q 17/04;**
A61K 2800/52

(86) International application number:
**PCT/EP2020/077552**

(87) International publication number:
**WO 2021/064116 (08.04.2021 Gazette 2021/14)**

(54) **COMPOSITIONS WITH POLYMERIC OR OLIGOMERIC HYDROXY PHENYL TRIAZINE UV FILTER**

ZUSAMMENSETZUNGEN MIT POLYMEREM ODER OLIGOMEREM HYDROXYPHENYLTRIAZIN-UV-FILTER

COMPOSITIONS CONTENANT UN FILTRE UV HYDROXY PHÉNYL TRIAZINE OLIGOMÈRE OU POLYMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2019 EP 19201163**

(43) Date of publication of application:
**10.08.2022 Bulletin 2022/32**

(73) Proprietor: **BASF SE**
**67056 Ludwigshafen am Rhein (DE)**

(72) Inventor: **EHLIS, Thomas**
**4133 Schweizerhalle (Muttenz) (CH)**

(74) Representative: **BASF IP Association**
**Carl-Bosch-Straße 38**
**67056 Ludwigshafen (DE)**

(56) References cited:
**WO-A1-03/004557**
**WO-A1-2009/080548**
**WO-A2-2004/104081**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 037 654 B1

## Description

The present invention relates to compositions comprising at least one polymeric or oligomeric hydroxy phenyl triazine UV filter and at least one additional UV filter. Furthermore, the present invention relates to the use of at least one polymeric or oligomeric hydroxy phenyl triazine UV filter for improving the photostability of a sunscreen composition comprising at least one additional UV filter, wherein the at least one further UV filter is selected from organic UV-A, UV-B, broad spectrum UV filter or a combination thereof.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter and broadband filters. These filters are added to sunscreen or cosmetic compositions. The UV filters are either organic or inorganic, particulate or non-particulate compounds, of which all have a high absorption efficacy in the UV-light range. In general, UV light can be divided into UV-A radiation (320 - 400 nm) and UV-B radiation (280 - 320 nm). Depending on the position of the absorption maxima, UV-filters are divided into UV-A and UV-B filters. In case an UV-filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

However, the UV filters known in the prior art, which are used in sunscreen or cosmetic compositions have certain disadvantages. In particular, it is referred to the disadvantage of certain UV filter, which tend to isomerize upon UV irradiation. As a result, those UV filters lose their protective properties and need to be stabilized in sunscreen or cosmetic compositions. Commonly used stabilizer with UV protective properties are for example octocrylene or certain photostabilizing agents. Accordingly, there is a need for sunscreen or cosmetic compositions providing an efficient UVA and UVB protection. Therefore, todays focus is towards eliminating as much of UV-A and UV-B light as possible. Accordingly, there is a need for sun care products exhibiting a high sun protection factor (SPF) and a high UV-A protection factor while being photostable.

WO 2014/004176 A1 discloses ultraviolet radiation absorbing polymer compositions comprising a polyacide monomer bearing a UV chromophore, which is reacted with a monoglyceride.

WO 03/004557 A1 relates to hydroxyphenyltriazine UV-absorber compounds for use in protecting plants in greenhouses and for protecting packaged foodstuffs.

Therefore, it has been an object of the present invention, to provide compositions for efficient UV-A and UV-B protection. In this connection, it has been another object of the present invention to provide efficient UV protective compositions, which are photostable. It has been another object of the present invention, to provide compositions, which are suitable for improving the photostability of sunscreen compositions.

With regard to the above objects, it is to be understood that the compositions according to the present invention provide for efficient UV-A and UV-B protection, wherein the UV-A and UV-B protection are defined by the sun protection factor and/or the UV-A protection factor of the compositions of the present invention.

It has surprisingly been found that at least one of these objects can be achieved by the composition according to the present invention.

In particular, the inventors of the present application found that the composition according to the present invention provides an efficient UV-A and UV-B protection by combining polymeric or oligomeric triazine UV filters with at least one additional UV filter, wherein the at least one further UV filter is selected from organic UV-A, UV-B, broad spectrum UV filter or a combination thereof.

Furthermore, it has surprisingly been found by the inventors of the present application that the composition according to the present invention can be used to improve the photostability of the sunscreen composition.

Thus, according to one embodiment, the present invention relates to a composition comprising at least one polymeric or oligomeric hydroxy phenyl triazine UV filter and at least one additional UV filter, wherein the at least one further UV filter is selected from organic UV-A, UV-B, broad spectrum UV filter or a combination thereof.

In a preferred embodiment of said composition, the at least one polymeric or oligomeric hydroxy phenyl triazine UV filter is a compound according to formula (I)

$$-[A\text{-}L\text{-}D\text{-}L]_x- \qquad (I)$$

in which

x  is a number from 1 to 50;

A  is a group of the formula (II)

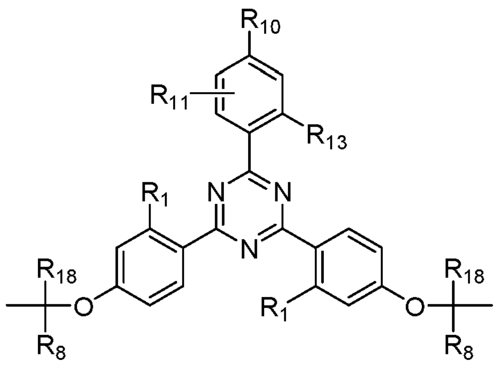

(II)

or has one of the meanings given for D, wherein formula (I) contains at least one A conforming to formula (II);

D is a divalent residue containing 2 to 60 carbon atoms comprising an aliphatic, cycloaliphatic or aromatic hydrocarbon, or said aliphatic residue substituted by OH or interrupted by O or both substituted by OH and interrupted by O; and in case that D bonds to the carbon atom of L, D also comprises methylene or a direct bond;

L stands for an ester linkage group;

the $R_1$ are independently of each other H, $OR_7$ or OH, with the proviso that at least one of $R_1$ or $R_{13}$ is OH;

the $R_7$ are independently of each other H, $C_1$-$C_{12}$-alkyl or a radical of formula (III)

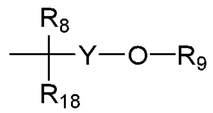

(III) ;

$R_8$ is H; $C_1C_{18}$-alkyl, wherein the $C_1$-$C_{18}$-alkyl group is unsubstituted or substituted with one or more same or different substituents selected from the group consisting of halogen, OH, and phenyl; $C_5$-$C_{12}$-cycloalkyl; $C_2$-$C_{18}$-alkenyl; $C_1C_{18}$-alkoxy; $C_3$-$C_{18}$-alkenyloxy; $C_7$-$C_{11}$-phenylalkyl; $C_7$-$C1_{1}$-alkylphenyl; $C_5$-$C_{12}$-cycloalkoxy; phenyl; or COOH;

Y is -CO-, or $C_1$-$C_{12}$-alkylene;

$R_9$ is, if Y is -CO-, $C_{20}$-$C_{60}$-alkyl unsubstituted or substituted by OH and/or interrupted by O, or is $C_{20}$-$C_{60}$-alkenyl, or is a group of formula (IV)

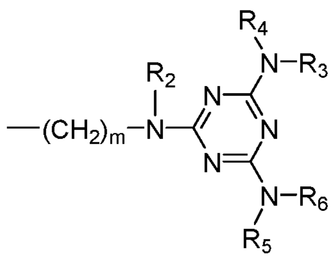

(IV)

,

wherein m is a number from 1 to 20;

or

| | |
|---|---|
| $R_9$ | is, if Y is alkylene, $C_{20}$-$C_{60}$-alkanoyl; |
| $R_2$, $R_3$, $R_4$, $R_5$ and $R_6$ | are independently of each other H, $C_1$-$C_{38}$-alkyl which is unsubstituted or substituted by OH or $C_1$-$C_8$-alkoxy; or $C_1$-$C_{38}$-alkyl which is interrupted by an oxygen atom or a N($C_1$-$C_{18}$)alkyl group; phenyl, or $C_7$-$C_{12}$-phenylalkyl which are unsubstituted or substituted by OH or $C_1$-$C_8$-alkyl; |
| $R_{10}$ | is H, $C_1$-$C_4$-alkyl, Cl, phenyl or a group $-OR_7$; |
| $R_{11}$ | is H or methyl; |
| $R_{13}$ | is H, methyl, OH or $OR_7$; |
| $R_{14}$ and $R_{15}$ | are independently H, $C_1$-$C_8$-alkyl, Cl, or a group $-OR_7$; |
| $R_{18}$ | is H or $C_1$-$C_8$-alkyl. |

In another preferred embodiment of said composition, the terminal groups of the polymer or oligomer according to formula (I) are selected from -L-D-$COOR_{12}$, -L-D-$OR_{12}$, or $OR_{12}$ if bonded to A or D; or -D-$COOR_{12}$, -D-$OR_{12}$ or -$R_{12}$ if bonded to L,
wherein
$R_{12}$ is H or $C_1$-$C_8$-alkyl.

In another preferred embodiment of said composition, L is an ester linkage group selected from -COO- or -OCO-.

In another preferred embodiment of said composition, the at least one polymeric or oligomeric hydroxy phenyl triazine UV filter is a compound according to formula (I)

$$-[A\text{-}L\text{-}D\text{-}L]_x- \qquad (I)$$

in which A is a group of formula (II), wherein

| | |
|---|---|
| $R_{10}$ | is a group $OR_7$; |
| $R_{11}$, $R_{13}$ | is H; |
| $R_1$ | is $OR_7$, |
| $R_8$, $R_{18}$ | is H, or $C_1$-$C_3$-alkyl; and wherein |
| $R_7$ | is H or $C_1$-$C_3$-alkyl. |

The at least one further UV filter is selected from organic UV-A, UV-B, broad spectrum UV filter or a combination thereof. In a more preferred embodiment of said composition, the at least one further organic UV-A, UV-B or broad spectrum UV filter is selected from the group consisting of micronized 5,6,5',6'-tetraphenyl-3-3'-(1,4-phenylene)bis(1,2,4-triazine) (INCI phenylene bis-diphenyltriazine), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 4-(tert.-butyl)-4'-methoxydi benzoyl methane (INCI butyl methoxydibenzoylmethane), diethylhexyl butamido triazone, 4,4',4"-(1,3,5-triazin-2,4,6-triyltriimino)tris-benzoic acid-tris(2-ethylhexyl)ester (INCI ethylhexyl triazone), bis(butylbenzoate) diaminotriazine aminopropylsiloxane, hexyl 2-[4-(diethylamino)-2-hydroxybenzoyl]benzoate (INCI diethylamino hydroxybenzoyl hexyl benzoate), 3,3,5-trimethyl-cyclohexyl salicylate (homosalate), (RS)-2-ethylhexyl-2-hydroxybenzoate (INCI ethylhexyl salicylate), 2-ethylhexyl 2-cyano-3,3-diphenyl-2-propenoate (INCI octocrylene), micronized 2,2'-methylene bis[6-(2H-benzotriazol-2-yl)-4-(1,1,3,3-tetramethylbutyl)phenol] (INCI methylene bis-benzotriazolyl tetramethylbutylphenol), 2-phenyl-1H-benzimidazole-5-sulfonic acid (INCI phenylbenzimidazole sulfonic acid), micronized 2,4,6-tris(biphenyl-4-yl)-1,3,5-triazine (INCI trisbiphenyl triazine), micronized (2-{4-[diethylamino-2-hydroxy-benzoyl)-benzoyl]-piperazine-1-carbonyl}-phenyl)-(4-diethylamino-2-hydroxy-phenyl)-methanone, 4-methoxycinnamic acid 2-ethylhexyl ester (INCI ethylhexyl methoxycinnamate), 2-(2H-benzotriazol-2-yl)-6-[(2-ethylhexyloxy)methyl]-4-methylphenol, benzylidene malonate 1, benzylidene malonate 2, benzylidene malonate 3, 2-propenoic acid 3-(4-methoxyphenyl)-2-methylphenyl ester (INCI 2-ethylhexyl methoxycinnamate), and combinations thereof.

In another more preferred embodiment of said composition, the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), titanium dioxide, (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxyl-phenyl}-6-(4-methoxyphenyl)-1,3,5 triazine (INCI bis-ethylhexyloxyphenol methoxyphenyl triazine) and combinations thereof.

In another more preferred embodiment of said composition, the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.

In another preferred embodiment of said composition, the composition is a sunscreen composition exhibiting a sun protection factor (SPF) of at least 30 and/or an UV-A protection factor of at least 10.

In another preferred embodiment of said composition, the UV-A protection is at least one third of the sun protection factor of the sunscreen composition, and/or shows a critical wavelength of at least 370 nm.

In another aspect, the present invention relates to the use of at least one polymeric or oligomeric hydroxy phenyl triazine UV filter for improving the photostability of a sunscreen composition comprising at least one additional UV filter, wherein the at least one further UV filter is selected from organic UV-A, UV-B, broad spectrum UV filter or a combination thereof.

In a preferred embodiment of said use, the at least one polymeric or oligomeric hydroxy phenyl triazine UV filter is a compound according to formula (I)

$$-[A\text{-}L\text{-}D\text{-}L]_x- \qquad (I)$$

in which

x is a number from 1 to 50;
A is a group of the formula (II)

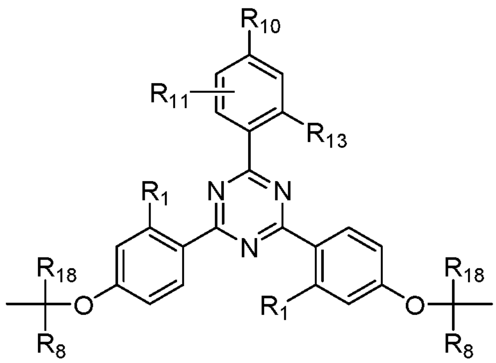
(II)

or has one of the meanings given for D, wherein formula (I) contains at least one A conforming to formula (II);

D is a divalent residue containing 2 to 60 carbon atoms comprising an aliphatic, cycloaliphatic or aromatic hydrocarbon, or said aliphatic residue substituted by OH or interrupted by O or both substituted by OH and interrupted by O; and in case that D bonds to the carbon atom of L, D also comprises methylene or a direct bond;
L stands for an ester linkage group;
the $R_1$ are independently of each other H, $OR_7$ or OH, with the proviso that at least one of $R_1$ or $R_{13}$ is OH;
the $R_7$ are independently of each other H, $C_1$-$C_{12}$-alkyl or a radical of formula (III)

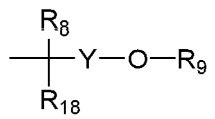
(III) .

$R_8$ is H; $C_1C_{18}$-alkyl, wherein the $C_1$-$C_{18}$-alkyl group is unsubstituted or substituted with one or more same or different substituents selected from the group consisting of halogen, OH, and phenyl; $C_5$-$C_{12}$-cycloalkyl; $C_2$-$C_{18}$-alkenyl; $C_1$-$C_{18}$-alkoxy; $C_3$-$C_{18}$-alkenyloxy; $C_7$-$C_{11}$-phenylalkyl; $C_7$-$C_{11}$-alkylphenyl; $C_5$-$C_{12}$-cycloalkoxy; phenyl; or COOH;
Y is -CO-, or $C_1$-$C_{12}$-alkylene;
$R_9$ is, if Y is -CO-, $C_{20}$-$C_{60}$-alkyl unsubstituted or substituted by OH and/or interrupted by O, or is $C_{20}$-$C_{60}$-alkenyl, or is a group of formula (IV)

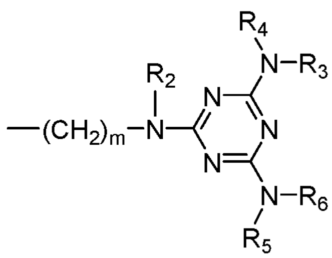
(IV)

,

wherein m is a number from 1 to 20;
or

$R_9$ is, if Y is alkylene, $C_{20}$-$C_{60}$-alkanoyl;

| | |
|---|---|
| $R_2$, $R_3$, $R_4$, $R_5$ and $R_6$ | are independently of each other H, $C_1$-$C_{38}$-alkyl which is unsubstituted or substituted by OH or $C_1$-$C_8$-alkoxy; or $C_1$-$C_{38}$-alkyl which is interrupted by an oxygen atom or a N($C_1$-$C_{18}$)alkyl group; phenyl, or $C_7$-$C_{12}$-phenylalkyl which are unsubstituted or substituted by OH or $C_1$-$C_8$-alkyl; |
| $R_{10}$ | is H, $C_1$-$C_4$-alkyl, Cl, phenyl or a group -$OR_7$; |
| $R_{11}$ | is H or methyl; |
| $R_{13}$ | is H, methyl, OH or $OR_7$; |
| $R_{14}$ and $R_{15}$ | are independently H, $C_1$-$C_8$-alkyl, Cl, or a group -$OR_7$; |
| $R_{18}$ | is H or $C_1$-$C_8$-alkyl. |

In another preferred embodiment of said use, the terminal groups of the polymer or oligomer according to formula (I) are selected from -L-D-$COOR_{12}$, -L-D-$OR_{12}$, or $OR_{12}$ if bonded to A or D; or -D-$COOR_{12}$, -D-$OR_{12}$ or -$R_{12}$ if bonded to L,
wherein
$R_{12}$ is H or $C_1$-$C_8$-alkyl.
In another preferred embodiment of said use, L is an ester linkage group selected from -COO- or -OCO-.
In another preferred embodiment of said use, the at least one polymeric or oligomeric hydroxy phenyl triazine UV filter is a compound according to formula (I)

$$-[A-L-D-L]_x- \qquad (I)$$

in which A is a group of formula (II), wherein

| | |
|---|---|
| $R_{10}$ | is a group $OR_7$; |
| $R_{11}$, $R_{13}$ | is H; |
| $R_1$ | is $OR_7$, |
| $R_8$, $R_{18}$ | is H, or $C_1$-$C_3$-alkyl; and wherein |
| $R_7$ | is H, or $C_1$-$C_3$-alkyl. |

In another preferred embodiment of said use, the at least one further UV filter is a UV-A, UV-B, a broad spectrum UV filter or a combination thereof.
In said use, the at least one further UV filter is selected from organic UV-A, UV-B, broad spectrum UV filter or a combination thereof.
In a more preferred embodiment of said use, the at least one further organic UV-A, UV-B or broad spectrum UV filter is selected from the group consisting of micronized 5,6,5',6'-tetraphenyl-3-3'-(1,4-phenylene)bis(1,2,4-triazine) (INCI phenylene bis-diphenyltriazine), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 4-(tert.-butyl)-4'-methoxydibenzoylmethane (INCI butyl methoxydibenzoylmethane), diethylhexyl butamido triazone, 4,4',4"-(1,3,5-triazin-2,4,6-triyltriimino)tris-benzoic acid-tris(2-ethylhexyl)ester (INCI ethylhexyl triazone), bis(butylbenzoate) diaminotriazine aminopropylsiloxane, hexyl 2-[4-(diethylamino)-2-hydroxybenzoyl]benzoate (INCI diethylamino hydroxybenzoyl hexyl benzoate), 3,3,5-trimethyl-cyclohexyl salicylate (homosalate), (RS)-2-ethylhexyl-2-hydroxybenzoate (INCI ethylhexyl salicylate), 2-ethylhexyl 2-cyano-3,3-diphenyl-2-propenoate (INCI octocrylene), micronized 2,2'-methylene bis[6-(2H-benzotriazol-2-yl)-4-(1,1,3,3-tetramethylbutyl)phenol] (INCI methylene bis-benzotriazolyl tetramethylbutylphenol), 2-phenyl-1H-benzimidazole-5-sulfonic acid (INCI phenylbenzimidazole sulfonic acid), micronized 2,4,6-tris(biphenyl-4-yl)-1,3,5-triazine (INCI trisbiphenyl triazine), micronized (2-{4-[diethylamino-2-hydroxy-benzoyl)-benzoyl]-piperazine-1-carbonyl}-phenyl)-(4-diethylamino-2-hydroxy-phenyl)-methanone, 4-methoxycinnamic acid 2-ethylhexyl ester (INCI ethylhexyl methoxycinnamate), 2-(2H-benzotriazol-2-yl)-6-[(2-ethylhexyloxy)methyl]-4-methylphenol, benzylidene malonate 1, benzylidene malonate 2, benzylidene malonate 3, 2-propenoic acid 3-(4-methoxyphenyl)-2-methylphenyl ester (INCI 2-ethylhexyl methoxycinnamate), and combinations thereof.
In another more preferred embodiment of said use, the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), titanium dioxide, (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.
In another more preferred embodiment of said use, the at least one further organic UV-A, UV-B or broad spectrum UV filter is selected from the group consisting of 4-methoxycinnamic acid 2-ethylhexyl ester (INCI ethylhexyl

methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.

In another preferred embodiment of said use, the composition is a sunscreen composition exhibiting a sun protection factor (SPF) of at least 30 and/or an UV-A protection factor of at least 10.

In another preferred embodiment of said use, the UV-A protection is at least one third of the sun protection factor of the sunscreen composition, and/or shows a critical wavelength of at least 370 nm.

The compounds according to formula I or precursors thereof can be prepared in analogy to one of the methods indicated in EP434608 or in the publication by H. Brunetti and C.E. Lüthi, Helv. Chim. Acta 55, 1566 (1972), by Friedel-Crafts addition of halotriazines with the corresponding phenols (see also US3118887, EP165608).

(Poly)esters of formula I are preferably prepared starting from tris-aryltriazines containing two carboxylic acid groups or suitable derivatives thereof such as acid chloride, anhydride or especially ester groups, or, alternatively, two reactable, preferably primary, OH groups. Such educts or their homologues are described, inter alia, in US4826978, US5736597, US5986233, and US5959008. Further educts of the same type, e.g. aliphatic, cycloaliphatic or aromatic dicarboxylic acids or derivatives thereof, or dialcohols, may be added. For esterification. the dicarboxylic educts are preferably reacted according to methods known in the art with suitable amounts, e.g.0.9 - 1.1 mol per mol dicarboxyl or equimolar amounts of a diol HO-D-OH, with or without additional dicarboxylic compounds present. Diol educts are correspondingly reacted with dicarboxylic acids, anhydrides acid chlorides or preferably esters, e.g. of formula $R_{12}$-O-DO-$R_{12}$. Preferred dicarboxylic acid educts include those based on oxalic, malonic, maleic, malic, fumaric, succinic, glutaric, adipinic, pimelic, suberic, azelaic, sebacic, phthalic, isophthalic, terephthalic, cyclohexyl dicarboxylic, glutaconic, itaconic, tartaric adic. Preferred diols include glycol, glycerin, various polyethylene glycols, or $\alpha,\omega$-dihydroxyalkanes of various chain lengths such as butanediol, pentanediol, hexanediol, heptanediol, octanediol, nonanediol, decanediol, undecanediol, dodecanediol, tridecanediol, pentadecanediol, octadecandiol, eicosanediol and mixtures thereof. The reaction can be carried out with or without addition of further components such as solvents (e.g. aliphatic alcohols, ethers, aromatic hydrocarbons, or halogenated hydrocarbons such as chlorobenzene or solvent mixtures) or catalysts, e.g. transesterification catalysts such as mineral or organic (Lewis or Broensted-type) acids or bases. In case that no additional solvent is used, a starting material such as the diol or a suitable ester of a dicarboxylic acid may be used in excess and serve simultaneously as a solvent. Temperature and pressure are usually not critical, thus, the reaction often is carried out at temperatures in the range of from -5 °C to 200 °C, e.g. between 10 °C and 170 °C and the pressure is close to one atmosphere, e.g. $10^4$ Pa to about $10^6$ Pa, with or without presence of oxygen, e.g. under nitrogen or argon.

Furthermore, the present invention also relates to an oligoester or polyester, which is obtained by reacting a tris-aryl-triazine of the formula V

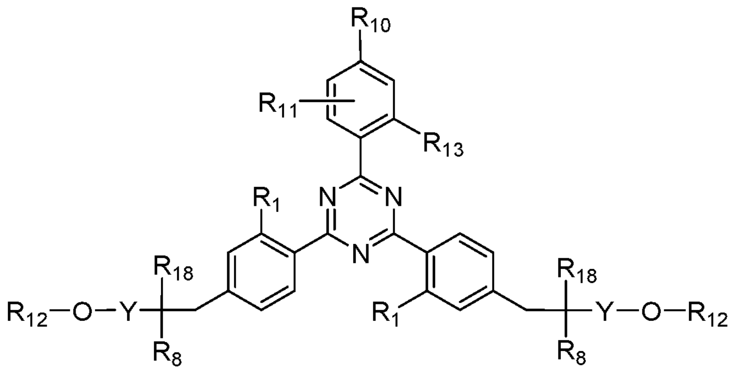

(V)

wherein all symbols are as defined below, provided that $R_{12}$ is H in case that Y is alkylene.

If Y is -CO-, with a diol HO-D-OH, optionally in presence of the formula $R_{12}$-O-T-O-$R_{12}$; and if Y is alkylene, with a diacid or diester $R_{12}$-O-T-O-$R_{12}$, optionally in presence of a diol of the formula HO-T-OH.

Before describing preferred embodiments of the present invention in detail, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of

±20%, preferably ±15%, more preferably ±10%, and even more preferably ±5%. It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is meant to also encompass a group, which preferably consists of these embodiments only.

Furthermore, the terms "first", "second", "third" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The term "sunscreen composition" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, or gels. The sunscreen composition may comprise one or more active agents, e.g., organic or inorganic UV filters, as well as other ingredients or additives, e.g., emulsifiers, emollients, viscosity regulators, stabilizers, preservatives, or fragrances.

The term "daily care composition" refers to any topical product, which reflects or absorbs certain parts of UV radiation and is used as an everyday care product for the human body, e.g., for face, body or hair. The daily care composition may comprise one or more active agents, e.g., organic or inorganic UV filters, as well as other ingredients or additives, e.g., emulsifiers, emollients, viscosity regulators, stabilizers, preservatives, or fragrances.

The term "sun protection factor (SPF)" as used herein indicates how well the skin is protected by a sunscreen composition mainly from UV-B radiation. In particular, the factor indicates how much longer the protected skin may be exposed to the sun without getting a sunburn in comparison to untreated skin. For example, if a sunscreen composition with an SPF of 15 is evenly applied to the skin of a person usually getting a sunburn after 10 minutes in the sun, the sunscreen allows the skilled person to stay in the sun 15 times longer. In other words, SPF 15 means that 1/15 of the burning UV radiation will reach the skin, assuming sunscreen is applied evenly at a thick dosage of 2 milligrams per square centimeter ($mg/cm^2$).

The term "critical wavelength" is defined as the wavelength at which the area under the UV protection curve (% protection versus wavelength) represents 90 % of the total area under the curve in the UV region (290-400 nm). For example, a critical wavelength of 370 nm indicates that the protection of the sunscreen composition is not limited to the wavelengths of UV-B, i.e. wavelengths from 290-320 nm, but extends to 370 nm in such a way that 90 % of the total area under the protective curve in the UV region are reached at 370 nm.

The term "UV-filter" as used herein refers to organic or inorganic compounds, which can absorb and/or reflect UV radiation caused by sunlight. UV-filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30 the UVA protection factor has to be at least 10.

The term "polymeric" refers to of, relating to, or consisting of a polymer. A polymer is a large molecule, or macromolecule consisting of a certain number of repeating units so called monomers.

The term "oligomeric" refers to of, relating to or consisting of an oligomer. An oligomer is a molecular complex of chemicals and consists of only a few, defined number of repeating units. In contrast thereto, a polymer as described above is in principle infinite.

The organic moieties mentioned in the above definitions of the variables are - like the term halogen - collective terms for individual listings of the individual group members. The prefix $C_n$-$C_m$ indicates in each case the possible number of carbon atoms in the group.

The term "halogen" denotes in each case fluorine, bromine, chlorine or iodine, in particular fluorine, chlorine or bromine.

The term "alkyl" as used herein denotes in each case a straight chain or branched alkyl group. Preferred alkyl groups include methyl, ethyl, n-propyl, iso-propyl, n-butyl, 2-butyl, iso-butyl, tert-butyl, 2-ethylbutyl, n-pentyl, iso-pentyl, 1-methylpentyl, 1 ,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl, dodecyl, 1,1,3,3,5,5-hexamethylhexyl, tridecyl, tetradecyl, pentadecyl, hexadecyl, heptadecyl and octadecyl. Examples for the $C_{20}$-$C_{60}$-alkyl are icosyl, henicosyl, docosyl, tricosyl, pentacosyl, heptacosyl, nonacosyl, triacontyl, dotriacontyl, tetracontyl, pentacontyl and hexacontyl. Furthermore, an alkyl interrupted by O, NH, N($C_1$-$C_{12}$)alkyl can gen-

erally comprise one or more nonadjacent heteroatoms. Preferably, a carbon atom of the alkyl chain bonds to not more than one heteroatom.

The term "alkoxy" as used herein denotes in each case a straight-chain or branched alkyl group, which is bonded via an oxygen group, having usually from 1 to 4 carbon atoms. Examples of alkoxy groups are methoxy, ethoxy, n-propoxy, iso-propoxy, n-butyloxy, 2-butyloxy, iso-butyloxy, tert.-butyloxy, and the like.

The term "alkenyl" as used herein denotes in each case an at least singly unsaturated hydrocarbon radical, i.e. a hydrocarbon radical having at least one carbon-carbon double bond. The alkenyl radicals preferably include allyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-octadec-2-enyl and n-octadec-4-enyl.

The term "alkenyloxy" refers to an alkenyl as defined above, which is attached to the remainder of the molecule via an oxygen atom.

The term "cycloalkyl" as used herein and in the cycloalkyl moieties of cycloalkoxy and cycloalkylthio denotes in each case a monocyclic cycloaliphatic radical having usually from 3 to 10 or from 3 to 6 carbon atoms, such as cyclopropyl, cyclobutyl, cyclopentyl, cyclohexyl, cycloheptyl, cyclooctyl, cyclononyl and cyclodecyl or cyclopropyl, cyclobutyl, cyclopentyl and cyclohexyl.

The term "phenylalkyl" as used herein denotes a phenyl group, which is bonded via an alkyl group to the remainder of the molecule, i.e. a phenyl-substituted alkyl. Preferred phenylalkyl groups are for example $C_7$-$C_{11}$-phenylalkyl, such as benzyl, α-methylbenzyl, α-ethylbenzyl, α,α-dimethylbenzyl, phenylethyl, phenylpropyl, phenylbutyl and phenylpentyl.

The term "alkylphenyl" as used herein denotes an alkyl group, which is bonded via a phenyl group to the remainder of the molecule, i.e. an alkyl-substituted phenyl group. For example, $C_7$-$C_{14}$-alkylphenyl embraces examples such as methylphenyl (tolyl), dimethylphenyl (xylyl), trimethylphenyl (mexityl), ethylphenyl, propylphenyl, butylphenyl, dibutylphenyl, pentylphenyl, hexylphenyl, heptylphenyl, and octylphenyl.

The term "cycloalkoxy" as used herein denotes in each case a monocyclic cycloaliphatic radical having usually from 3 to 10 or from 3 to 6 carbon atoms which is bonded to the remainder of the molecule via oxygen.

The term "alkylene" refers to alkyl as defined above, which is bonded to the remainder of the molecule, via two atoms, preferably via two carbon atoms, of the respective group, so that they represent a linker between two moieties of the molecule, e.g. methylene or ethylene.

The term "aliphatic" can refer to any non-aromatic hydrocarbon group, wherein the constituent carbon atoms can be straight chain, branched chain, or cyclic and/or wherein heteroatoms can be bond to the carbon chain. Furthermore, these aliphatic groups can be substituted by one or more, same or different substituents.

The term "aromatic" refers to fully unsaturated hydrocarbons with a cyclic, planar molecule with a ring of resonance bonds.

The term "alkanoyl" is a different term for an "acyl" group, which refers to any carbonyl group bonded via the carbon atom of the carbonyl group (-CO) to the remainder of the molecule.

The term "terminal group" as used herein, relates to a chemical group or moiety, which is attached to the remainder of the molecule via a single atom.

The term "linkage group" relates to a group, preferably an alkyl group, which is substituted or unsubstituted with one or more substituents. Said group connects to moieties of a compound or a molecule with each other.

The term "divalent" refers to the two-fold binding affinity of an atom. The binding affinity or also valence of an element is a measure of its combining power with other atoms when it forms chemical compounds or molecules.

The term "micronized" refers to a substance or a compound, which is reduced to a fine powder, the particles of which are measured in microns in diameter. The term "micron" relates to a unit of length, which is equal to $10^6$ meter.

The term "photostability" refers to the ability of a UV filter or any other molecule, which is exposed to sunlight, to stay stable upon irradiation. In particular, this means that the compound does not undergo a degradation process upon UV radiation.

Preferred embodiments regarding the composition according to the present application as well as the use of the polymeric or oligomeric hydroxyphenyl triazine UV filter are described hereinafter. It is to be understood that the preferred embodiments of the invention are preferred alone or in combination with each other.

As indicated above, the present invention relates in one embodiment to a composition comprising at least one polymeric or oligomeric hydroxy phenyl triazine UV filter and at least one additional UV filter.

Preferred embodiments regarding the compounds of formula I and II, which are relevant for all aspects of the invention, are defined hereinafter.

In one embodiment of the present invention, the at least one polymeric or oligomeric hydroxy phenyl triazine UV filter is a compound according to formula (I)

$$-[A\text{-}L\text{-}D\text{-}L]_x- \qquad (I)$$

in which

x is a number from 1 to 50;
A is a group of the formula (II)

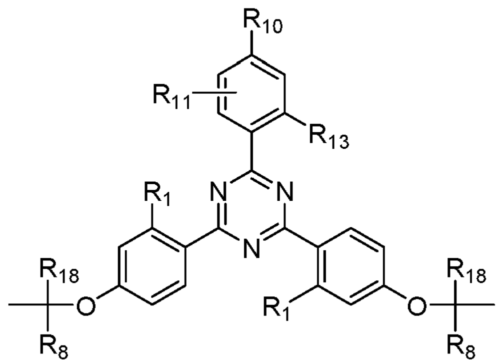

(II)

or has one of the meanings given for D, wherein formula (I) contains at least one A conforming to formula (II);

D is a divalent residue containing 2 to 60 carbon atoms comprising an aliphatic, cycloaliphatic or aromatic hydrocarbon, or said aliphatic residue substituted by OH or interrupted by O or both substituted by OH and interrupted by O; and in case that D bonds to the carbon atom of L, D also comprises methylene or a direct bond;
L stands for an ester linkage group;
the $R_1$ are independently of each other H, $OR_7$ or OH, with the proviso that at least one of $R_1$ or $R_{13}$ is OH;
the $R_7$ are independently of each other H, $C_1$-$C_{12}$-alkyl or a radical of formula (III)

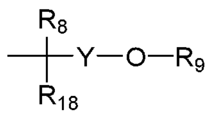

(III);

$R_8$ is H; $C_1C_{18}$-alkyl, wherein the $C_1$-$C_{18}$-alkyl group is unsubstituted or substituted with one or more same or different substituents selected from the group consisting of halogen, OH, and phenyl; $C_5$-$C_{12}$-cycloalkyl; $C_2$-$C_{18}$-alkenyl; $C_1$-$C_{18}$-alkoxy; $C_3$-$C_{18}$-alkenyloxy; $C_7$-$C_{11}$-phenylalkyl; $C_7$-$C_{11}$-alkylphenyl; $C_5$-$C_{12}$-cycloalkoxy; phenyl; or COOH;
Y is -CO-, or $C_1$-$C_{12}$-alkylene;
$R_9$ is, if Y is -CO-, $C_{20}$-$C_{60}$-alkyl unsubstituted or substituted by OH and/or interrupted by O, or is $C_{20}$-$C_{60}$-alkenyl, or is a group of formula (IV)

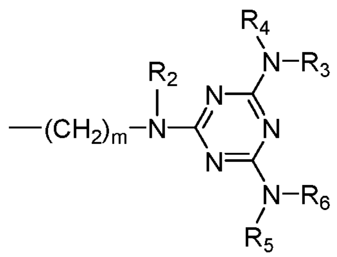

(IV)

,

wherein m is a number from 1 to 20;
or

$R_9$ is, if Y is alkylene, $C_{20}$-$C_{60}$-alkanoyl;
$R_2$, $R_3$, $R_4$, $R_5$ and $R_6$ are independently of each other H, $C_1$-$C_{38}$-alkyl which is unsubstituted or substituted by OH or $C_1$-$C_8$-alkoxy; or
$C_1$-$C_{38}$-alkyl which is interrupted by an oxygen atom or a $N(C_1$-$C_{18})$alkyl group; phenyl, or $C_7$-$C_{12}$-phenylalkyl which are unsubstituted or substituted by OH or $C_1$-$C_8$-alkyl;

$R_{10}$ is H, $C_1$-$C_4$-alkyl, Cl, phenyl or a group -$OR_7$;
$R_{11}$ is H or methyl;
$R_{13}$ is H, methyl, OH or $OR_7$;
$R_{14}$ and $R_{15}$ are independently H, $C_1$-$C_8$-alkyl, Cl, or a group -$OR_7$;
$R_{18}$ is H or CrCa-alkyl.

In a preferred embodiment of the invention, the compound of formula I is

-[A-L-D-L]$_x$- (I),

wherein

the $R_7$ are independently of each other H, $C_1$-$C_{12}$-alkyl or a radical of formula (III)

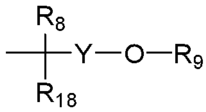
(III),

wherein $R_8$ is H or $C_1$-$C_8$-alkyl;
Y is -CO- or $C_1$-$C_2$-alkylene;
$R_9$ if Y is -CO-, is $C_{20}$-$C_{60}$-alkyl, $C_{20}$-$C_{60}$-alkyl substituted by OH and/or interrupted by O, or is a group of formula (IV)

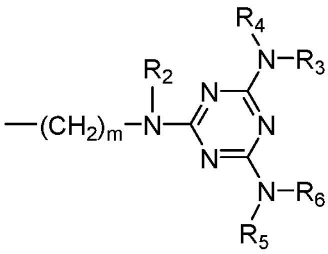
(IV) ,

wherein m is a number from 2 to 12;
$R_9$, if Y is alkylene, is $C_{20}$-$C_{60}$-alkanoyl;
$R_2$, $R_3$, $R_4$, $R_5$ and $R_6$ are independently of each other H, $C_1$-$C_{18}$-alkyl or $C_4$-$C_{12}$-hydroxyalkyl or $C_4$-$C_{12}$-alkoxyalkyl.

In another preferred embodiment of the present invention, in the compound of formula II $R_{10}$ is H, $C_1$-$C_4$-alkyl or a group -$OR_7$;

$R_{11}$ and $R_{18}$ independently are H or methyl;
$R_{13}$ is H, OH or methyl.

In a more preferred embodiment of the present invention,

$R_8$ is H or $C_1$-$C_4$-alkyl;
$R_{10}$ is H, methyl or a group -$OR_7$;
$R_{11}$ and $R_{18}$ are H;
$R_{13}$ is H, OH or methyl.
the $R_7$ are H or methyl or a radical of formula (III)

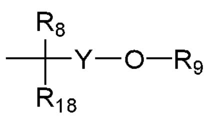
(III) .

In a particularly preferred embodiment of the present invention, in the compound according to formula II $R_1$ is OH.
In another preferred embodiment of the present invention, terminal groups of the polymer or oligomer according to formula I usually are selected from -L-D-COOR$_{12}$, -L-D-OR$_{12}$, or -OR$_{12}$ if bonded to A or D; or -D-COOR$_{12}$, -D-OR$_{12}$, or -R$_{12}$ if bonded to L. In connection with the above preferred embodiment, it is to be understood that $R_{12}$ is H or $C_1$-$C_8$-alkyl.
In this connection, it is to be understood that the ester linkage group L is -COO- or -OCO-.
In a preferred embodiment of the present invention the ester of formula I may conform to formula Ia

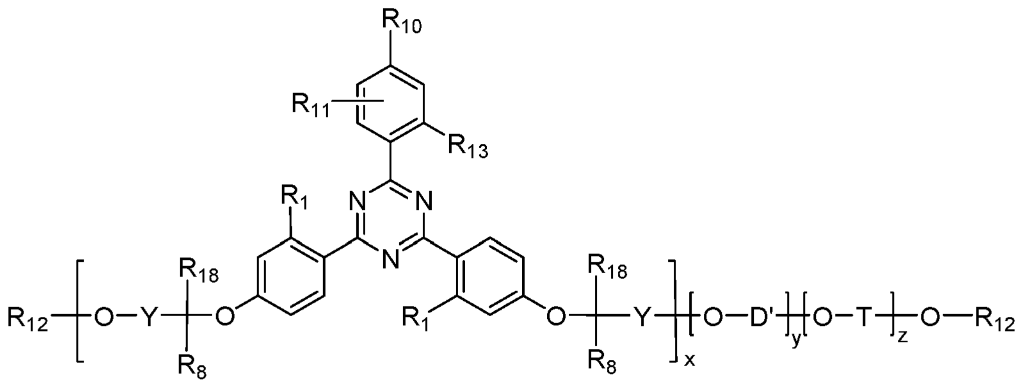

Ia,

in which

x is a number from 1 to 20;
the number y is at least 1 and ranges from (x+z-1) to (x+z+1);
z is a number from 0 to 20; and
$R_{12}$ is H or $C_1$-$C_8$-alkyl;
$R_{18}$ is H or $C_1$-$C_8$-alkyl;
Y is -CO- or $C_1$-$C_{12}$-alkylene;
if Y is -CO-, D' is $C_2$-$C_{38}$-alkylene or $C_4$-$C_{60}$-alkylene interrupted by O; and T is the divalent acyl residue of an aliphatic, cycloaliphatic or aromatic dicarboxylic acid of 2 to 12 carbon atoms;
if Y is alkylene, D' is the divalent acyl residue of an aliphatic, cycloaliphatic or aromatic dicarboxylic acid of 2 to 12 carbon atoms; and T is $C_2$-$C_{38}$-alkylene or $C_4$-$C_{60}$-alkylene interrupted by O. In connection with the above preferred embodiment, it is to be understood that all other symbols are as defined above.

In a more preferred embodiment of the present invention, in the oligo- or polyester of formula Ia, each of the divalent structural units identified by the indices x and z bonds to the structural unit -O-D'- identified by the index y, and/or to an end group $R_{12}$ or $OR_{12}$.
In another preferred embodiment of the present invention, the ester of formula I may also conform to formula Ib

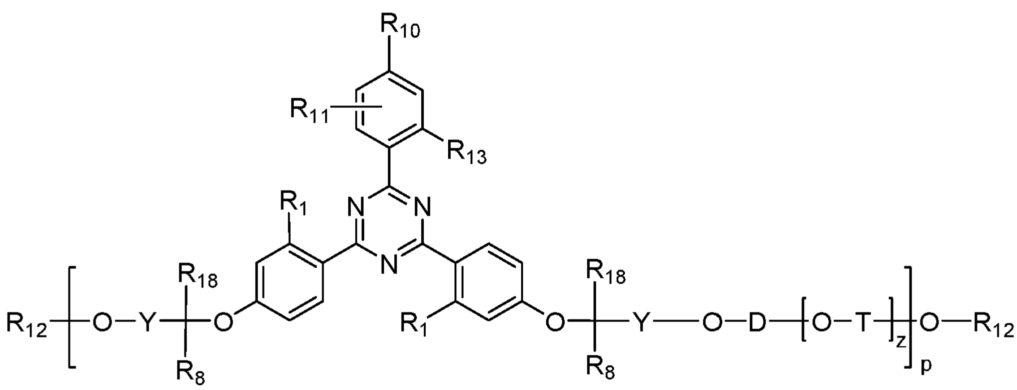

Ib,

wherein

p ranges from 1 to 12, especially from 2 to 8;

z is 0 or 1. In connection with the above preferred embodiment, it is to be understood that D corresponds to D' as defined above. Furthermore, it is to be understood that all other symbols are as defined above.

In connection with the compounds according to formula Ia, the moiety of formula

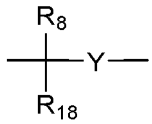

is most preferably $-CH(CH_3)-CO-$ or $-CH_2CO-$ with Y being CO, or is $-CH(CH_3)-CH_2-$ or $-CH_2CH_2-$ with Y being methylene.

In another preferred embodiment of the present invention, formula (III) conforms to formula

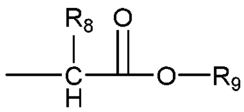

,

with $R_8$ being H or $C_1$-$C_8$-alkyl, especially methyl.

In a particularly preferred embodiment of the present invention, compounds of formula I usually are of the type heteropolyester comprising two classes of structural units, one derived from dicarboxylic acids and the other from diols. If in formula I the moiety L-A-L is a residue of a dicarboxylic acid, -D- is the residue of a divalent alcohol; if in formula I the moiety L-D-L is a residue of a dicarboxylic acid, -A- is the residue of a divalent alcohol.

In another particularly preferred embodiment of the present invention, L-D-L as a divalent acyl residue conforms to the formula -OCO-D-COO-, D' or T as a divalent acyl residue conform to the formula -CO-T'-CO-, wherein D or T' is, for example a direct bond, $C_1$-$C_{60}$-alkylene, $C_2$-$C_{10}$-alkenylene, phenylene, naphthylene, $C_5$-$C_8$-cycloalkylene, $C_2$-$C_4$-alkylene or alkenylene interrupted by O or cyclohexylene or phenylene, $C_1$-$C_{12}$-alkylene substituted by OH. More preferred D or T' in this meaning are phenylene, cyclohexylene, $C_2$-$C_{10}$-alkylene, or $C_2$-$C_{10}$-alkylene substituted by OH.

In another preferred embodiment of the present invention, D' or T as a diol residue conform to formula -O-T'-O-; D or T' in this meaning are, for example, $C_2$-$C_{60}$-alkylene, $C_2$-$C_{10}$-alkenylene, $C_5$-$C_8$-cycloalkylene, $C_4$-$C_{60}$-alkylene or alkenylene interrupted by O, cyclohexylene and/or phenylene. More preferred D or T' in this meaning are $C_2$-$C_{24}$-alkylene, or $C_4$-$C_{60}$-alkylene interrupted by O.

In another preferred embodiment of the present invention, in the compounds of formula I, x is preferably from the range 2-50, more preferably from the range 2-20, especially 4-12. In the compounds of formula Ia, each of x and y are preferably from the range 2-16, more preferably from the range 4-12; z is preferably ranging from 0-12. Of specific technical interest are compounds of formula Ia wherein z is 0.

In another preferred embodiment of the present invention, oligomeric or polymeric esters of then invention such as those of formula I usually have a molecular weight within the range 1000 to 50000 g/mol, more preferably 1500 to 20000 g/mol, most preferably 2000 to 10000 g/mol (number average Mn as determined by gel permeation chromatography GPC).

In a particularly preferred embodiment of the present invention, a compound according to formula I is preferred, wherein $R_8$ is $C_1$-$C_{18}$-alkyl, more preferably $C_1$-$C_8$-alkyl, in particular methyl. Furthermore, it is to be understood that the following preferences regarding $R_{18}$, n and p are part of the invention.

$R_{18}$ is preferably H, or methyl, especially H.

n is preferably 2-24, more preferably 4-16 and most preferably 4-12.

p is preferably 2-12 and more preferably 2-8.

In another particularly preferred embodiment of the present invention,
$R_9$ as $C_{20}$-$C_{60}$-alkyl substituted by OH preferably carries only one OH group in $\omega$ position. Preferred is a compound wherein $R_9$ is $C_{20}$-$C_{60}$-alkyl, more preferred $C_{26}$-$C_{52}$-alkyl and in particular $R_9$ is a mixture of alkyl with a range from 20 to 40 carbon atoms, with the mean value being around 32 carbon atoms.

The high alkyl groups of $R_9$ may also have a certain molecular distribution around their main component. Ranges may for example be from 22-26, 28-32 or 34-38 carbon atoms. It is however also possible that broader ranges are used such as for example from 20 to 40, from 30 to 50 or from 40 to 60 carbon atoms.

Commercially available alcohols for preparing the compounds according to formula (I) or (II) may contain small amounts of alkyl chains below $C_{20}$. Therefore, mixtures of compounds wherein $R_9$ is a mixture containing up to 10% of alkyl chains below 20 carbon atoms and 90 to 100% of alkyl chains between 20 and 60, particularly between 20 and 40 carbon atoms are also subject of the invention. Percentage is weight percent, based on the total mixture.

In another preferred embodiment of the present invention, if $R_9$ is a group of formula (IV), $R_2$ is preferably H

or $C_1$-$C_4$-alkyl;

$R_3$, $R_4$, $R_5$ and $R_6$ are preferably H or $C_1$-$C_{38}$-alkyl, more preferably $C_4$-$C_{24}$-alkyl and in particular $C_4$-$C_{12}$-alkyl.

In another preferred embodiment of the present invention, the at least one polymeric or oligomeric hydroxy phenyl triazine UV filter is a compound according to formula I

$$-[A\text{-}L\text{-}D\text{-}L]_x- \qquad (I).$$

In connection with the above preferred embodiment it is to be understood that A in the compound of formula I preferably comprises a group according to formula II

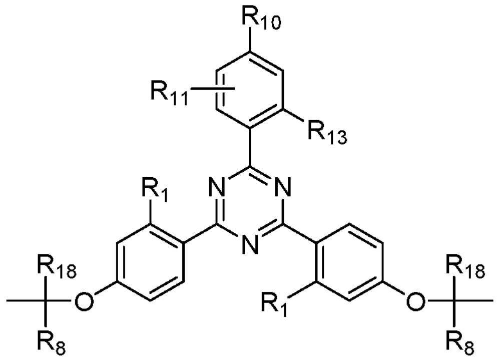

(II) ,

wherein

$R_{10}$ is a group $OR_7$
$R_{11}$, $R_{13}$ is H;
$R_1$ is $OR_7$;
$R_8$, $R_{18}$ is H, or $C_1$-$C_3$-alkyl.

In connection with the above particularly preferred embodiment, it is to be understood that $R_7$ is H, or $C_1$-$C_3$-alkyl, preferably $R_7$ is H or methyl.

In a particularly preferred embodiment of the present invention, the at least one polymeric or oligomeric hydroxy phenyl triazine UV filter refers to the following specific compounds.

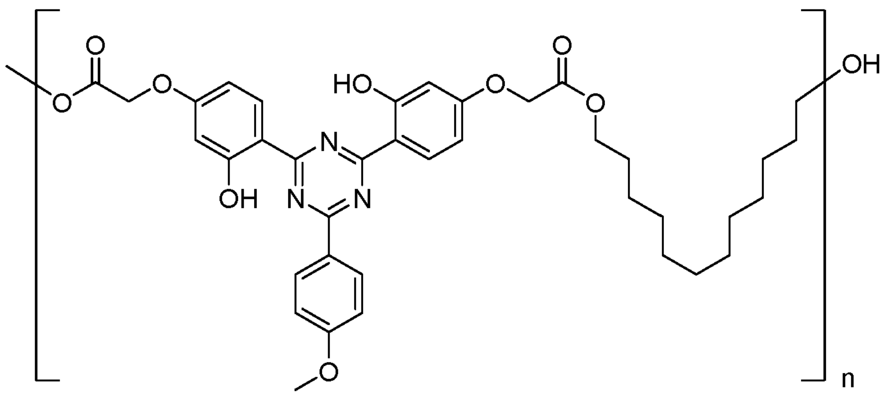

compound 1

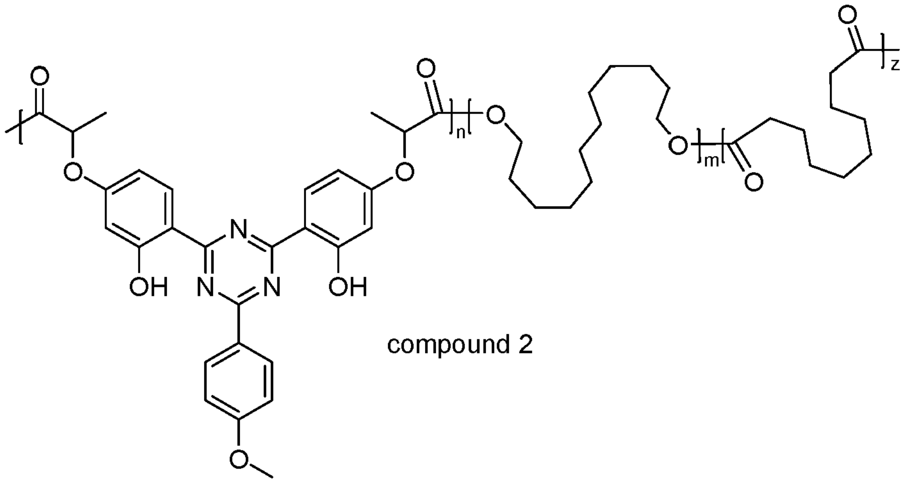

compound 2

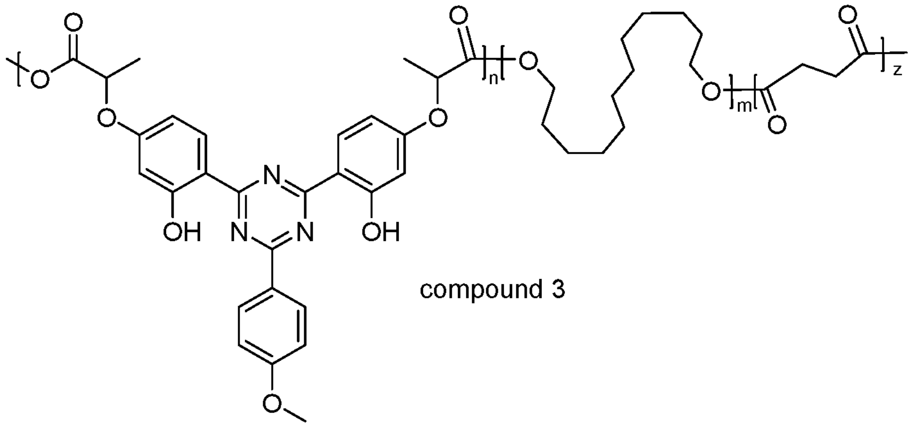

compound 3

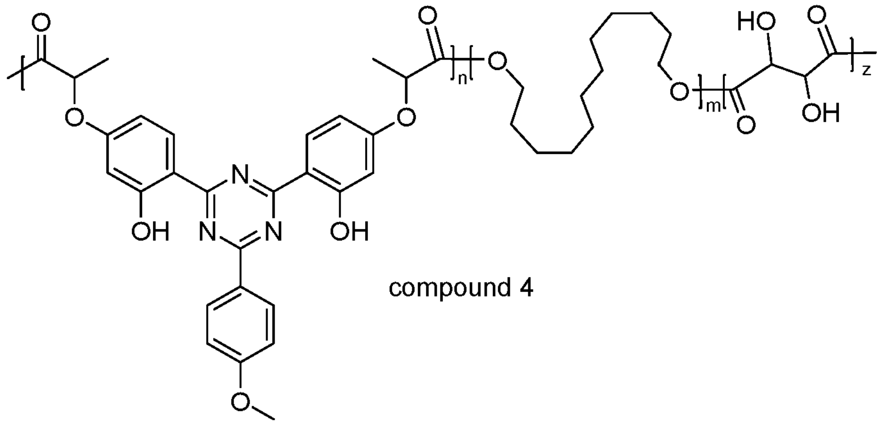

compound 4

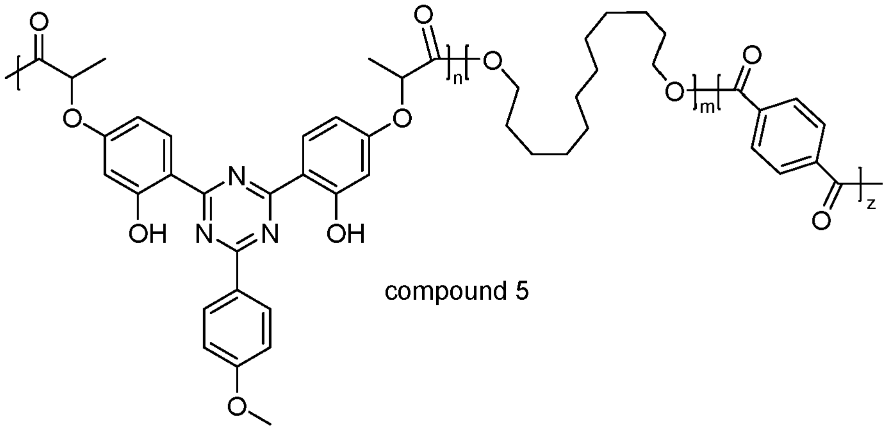

compound 5

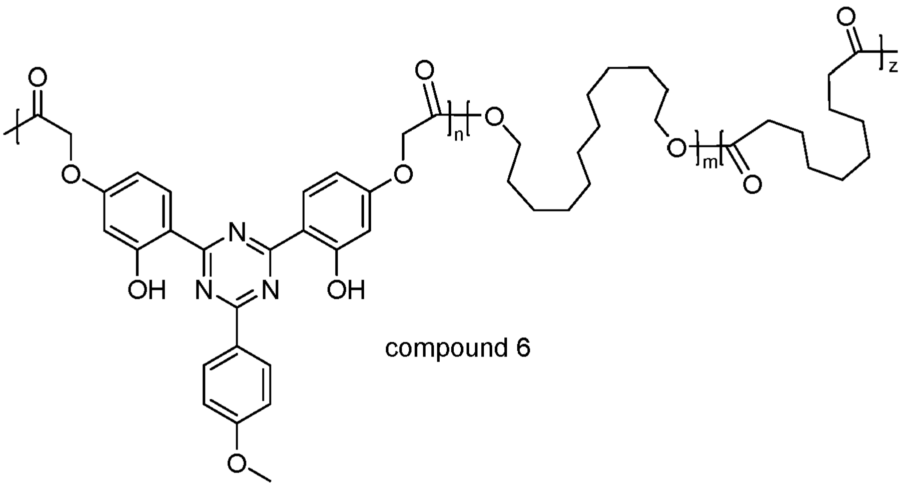

compound 6

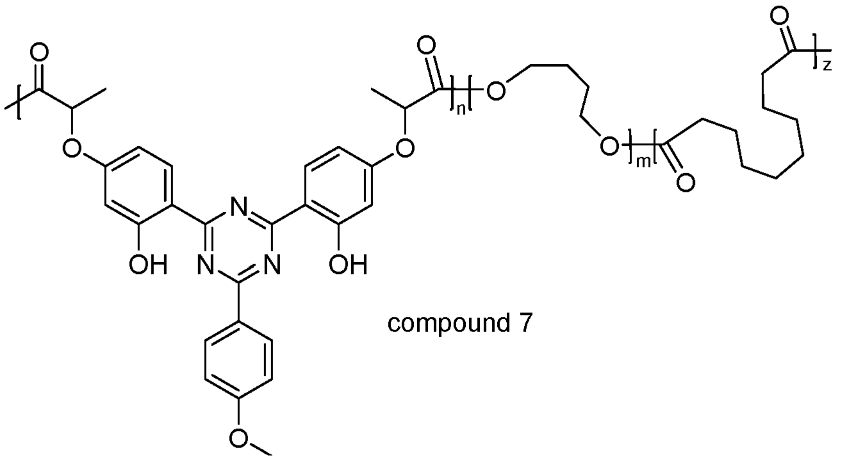

compound 7

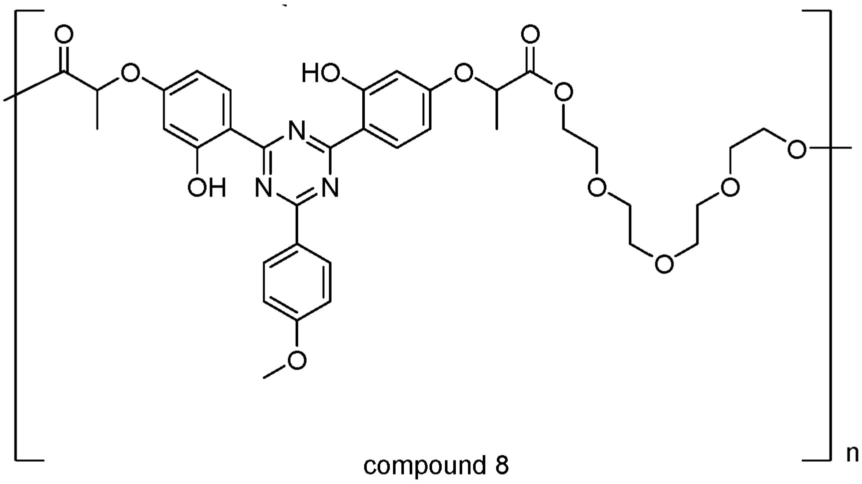

compound 8

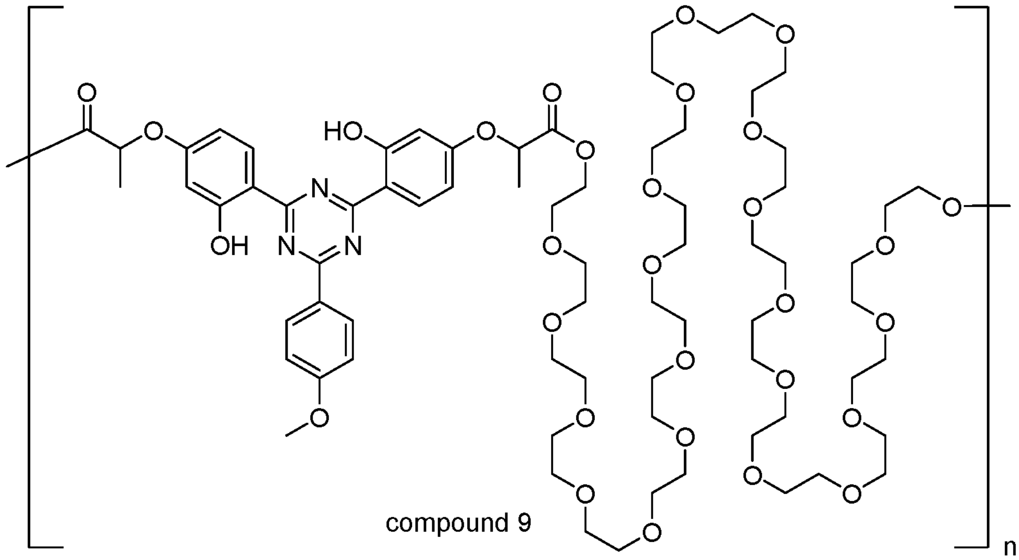

compound 9

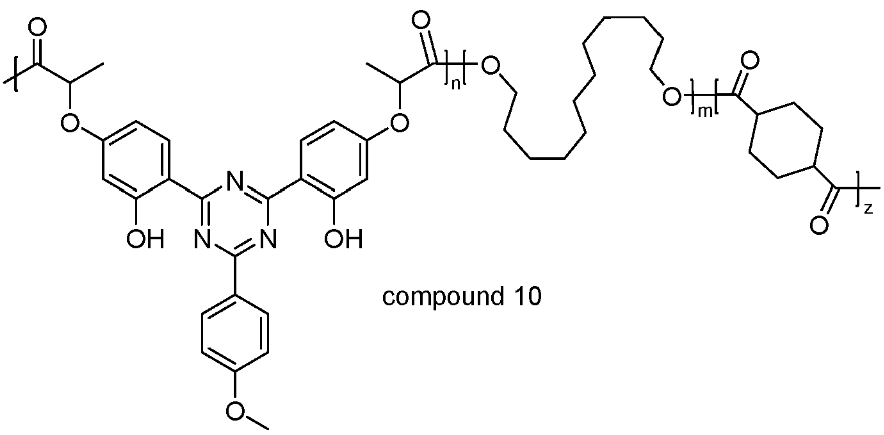

compound 10

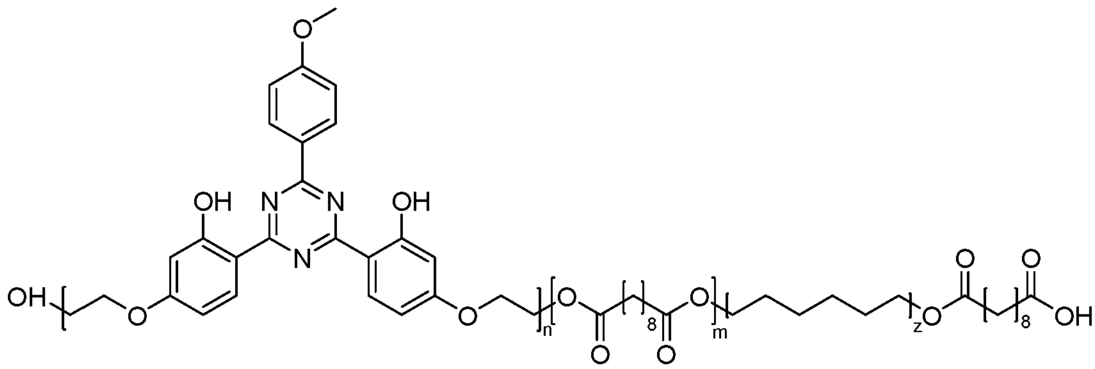

compound 11

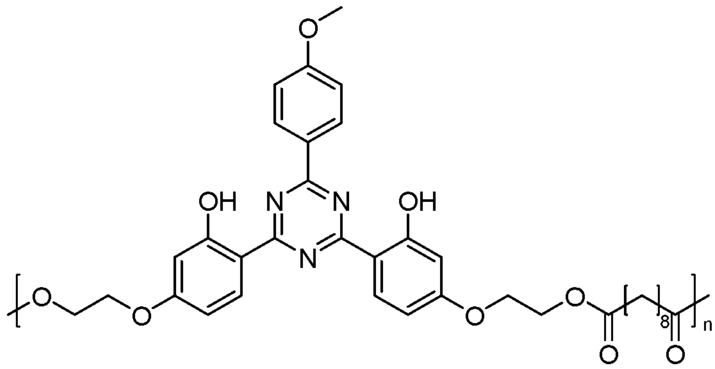

compound 12

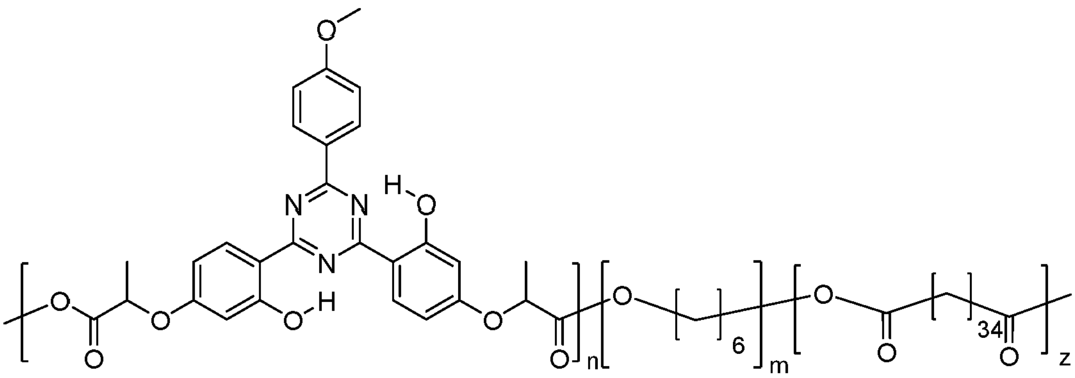

compound 13

n:m:z=0.6:1:0.4

In connection with the present invention, in particular in connection with the compounds according to formula I as defined above, the following preferences regarding the UV filter and the composition, i.e. the sunscreen composition are relevant in connection with the above listed embodiments of the invention.

In one embodiment of the present invention, the composition comprises at least one polymeric or oligomeric hydroxy phenyl triazine UV filter according to formula I as defined above and at least one additional UV filter, wherein the at least one further UV filter is selected from organic UV-A, UV-B, broad spectrum UV filter or a combination thereof. In this context, at least one further, or at least one additional organic UV filter means preferably from 1 to 3 additional organic UV filter, or more than 4 additional organic UV filter.

In another preferred embodiment of the present invention, the at least one further organic UV-A, UV-B or broad spectrum UV filter is selected from the group consisting of micronized 5,6,5',6'-tetraphenyl-3-3'-(1,4-phenylene)bis(1,2,4-triazine) (INCI phenylene bis-diphenyltriazine), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 4-(tert.-butyl)-4'-methoxydi benzoyl methane (INCI

butyl methoxydibenzoylmethane), diethylhexyl butamido triazone, 4,4',4"-(1,3,5-triazin-2,4,6-triyltriimino)tris-benzoic acid-tris(2-ethylhexyl)ester (INCI ethylhexyl triazone), bis(butylbenzoate) diaminotriazine aminopropylsiloxane, hexyl 2-[4-(diethylamino)-2-hydroxybenzoyl]benzoate (INCI diethylamino hydroxybenzoyl hexyl benzoate), 3,3,5-trimethyl-cyclohexyl salicylate (homosalate), (RS)-2-ethylhexyl-2-hydroxybenzoate (INCI ethylhexyl salicylate), 2-ethylhexyl 2-cyano-3,3-diphenyl-2-propenoate (INCI octocrylene), micronized 2,2'-methylene bis[6-(2H-benzotriazol-2-yl)-4-(1,1,3,3-tetramethylbutyl)phenol] (INCI methylene bis-benzotriazolyl tetramethylbutylphenol), 2-phenyl-1H-benzimidazole-5-sulfonic acid (INCI phenylbenzimidazole sulfonic acid), micronized 2,4,6-tris(biphenyl-4-yl)-1,3,5-triazine (INCI trisbiphenyl triazine), micronized (2-{4-[diethylamino-2-hydroxy-benzoyl)-benzoyl]-piperazine-1-carbonyl}-phenyl)-(4-diethylamino-2-hydroxy-phenyl)-methanone, 4-methoxycinnamic acid 2-ethylhexyl ester (INCI ethylhexyl methoxycinnamate), 2-(2H-benzotriazol-2-yl)-6-[(2-ethylhexyloxy)methyl]-4-methylphenol, benzylidene malonate 1, benzylidene malonate 2, benzylidene malonate 3, 2-propenoic acid 3-(4-methoxyphenyl)-2-methylphenyl ester (INCI 2-ethylhexyl methoxycinnamate), and combinations thereof.

In connection with the at least one further organic UV-A, UV-B or broad spectrum filter and with regard to the at least one oligomeric or polymeric hydroxy phenyl triazine UV filter according to formula I as defined above, preference is given to the following combinations compiled in Table 2 below. In connection with the below combinations of the oligomeric or polymeric hydroxy phenyl triazine UV filter and at least one further UV-A, UV-B or broad spectrum filter, the oligomeric or polymeric triazine UV filter is abbreviated to display PHPT.

In connection with the at least one further organic UV-A, UV-B or broad spectrum UV filter the following abbreviations as outlined in Table 1 are relevant.

Table 1

| | |
|---|---|
| PBDT | micronized phenylene bis diphenyltriazine |
| BMBM | butyl methoxydibenzoylmethane |
| DBT | diethylhexyl butamido triazone |
| EHT | ethylhexyl triazone |
| EHMC | ethylhexyl methoxy cinnamate |
| BBDAPT | bis(butylbenzoate) diaminotriazine aminopropylsiloxane |
| DHHB | diethylamino hydroxy benzoyl hexyl benzoate |
| EHS | ethylhexyl salicylate |
| HMS | homosalate |
| OCR | octocrylene |
| MBBT | micronized methylene bis-benzotriazolyl tetramethylbutylphenol |
| PBSA | phenylbenzimidazole sulfonic acid |
| | |
| TBT | micronized tris-biphenyl triazine |
| DHHM | micronized (2-{4-[2-(4-diethylamino-2-hydroxy-benzoyl)-benzoyl]-piperazine-1-carbonyl}-phenyl)-(4-diethylamino-2-hydroxy-phenyl)-methanone |
| BEMT* | Tinosorb S; Tinosorb S Aqua (bis-ethylhexyloxyphenol methoxyphenyl triazine encapsulated in a polymer matrix (as described in IP.com Journal 2009, 9(1B), 17)) |
| BTZOM | 2-(2H-benzotriazol-2-yl)-6-[(2-ethylhexyloxy)methyl]-4-methylphenol |
| MBM1 | 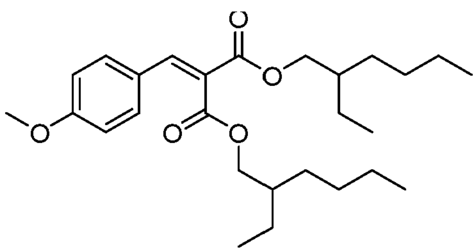 benzylidene malonate 1 |

(continued)

| | |
|---|---|
| MBM2 | 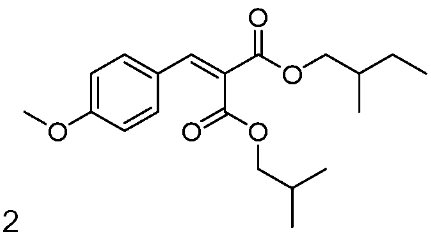 benzylidene malonate 2 |
| MBM3 | 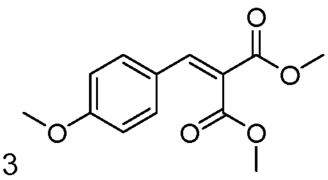 benzylidene malonate 3 |
| MPMPA | 2-propenoic acid, 3-(4-methoxyphenyl)-, 2-methylphenyl ester, (2E)- |

In connection with the composition, comprising PHPT and at least one additional UV filter, preference is given to the combinations as listed in Table 2. In this connection, it is to be understood that preferred compositions correspond in each case to one row of Table 2. Furthermore, it is to be understood, if more than 4 additional UV filter are present in the composition, two or more UV filter referring to component (b) of column 4 can be selected.

Further, it is to be understood that PHPT refers to the particularly preferred compounds 1 to 13 as listed above.

Table 2

| | UV filter component (a) | UV filter component (b) | | | |
|---|---|---|---|---|---|
| | | 1 | 2 | 3 | ≥4 |
| Combination with one additional UV filter | PHPT | PBDT | | | |
| | | BEMT* | | | |
| | | BMBM | | | |
| | | DBT | | | |
| | | EHT | | | |
| | | BBDAPT | | | |
| | | DHHB | | | |
| | | EHS | | | |
| | | HMS | | | |
| | | OCR | | | |
| | | MBBT | | | |
| | | PBSA | | | |
| | | | | | |
| | | TBT | | | |
| | | DHHM | | | |
| | | BTZOM | | | |
| | | MBM1 | | | |
| | | MBM2 | | | |
| | | MBM3 | | | |

(continued)

| | UV filter component (a) | UV filter component (b) | | | |
|---|---|---|---|---|---|
| | | 1 | 2 | 3 | ≥4 |
| | | MPMPA | | | |
| | | | | | |
| | | EHMC | | | |
| Combination with two additional UV filter | PHPT | MBBT | BEMT*<br>DHHB<br>BMBM | | |
| | | BEMT* | DHHB<br>BMBM | | |
| | | DHHB | BMBM | | |
| | | EHT BBDAPT | MBBT<br>BEMT*<br>DHHB<br>BMBM | | |
| Combination with three additional UV filter | PHPT | MBBT | BEMT*<br>DHHB<br>BMBM | EHT<br>BBDAPT | |
| | | BEMT* | DHHB<br>BMBM | EHT<br>BBDAPT | |
| | | DHHB | BMBM | EHT<br>BBDAPT | |
| Combination with more than four additional UV filter | PHPT | BEMT* | MBBT<br>DHHB<br>BMBM | EHT | EHS<br>EHMC<br>TBT<br>OCR<br>DBT<br>PBSA<br>HMS<br>DHHM<br>BBDAPT |
| | | MBBT | DHHB<br>BMBM | EHT<br>BBDAPT | EHS<br>EHMC<br>TBT<br>OCR<br>DBT<br>PBSA<br>HMS<br>DHHM |

(continued)

| | UV filter component (a) | UV filter component (b) | | | |
|---|---|---|---|---|---|
| | | 1 | 2 | 3 | ≥4 |
| | | EHT | DHHB<br>BMBM | BBDAPT | EHS<br>EHMC<br>TBT<br>OCR<br>DBT<br>PBSA<br>HMS<br>DHHM |

In a particularly preferred embodiment of the present invention, the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), titanium dioxide, (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.

In another particularly preferred embodiment of the present invention, the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.

In one embodiment of the present invention, the composition is a sunscreen composition exhibiting a sun protection factor (SPF) of at least 30.

In another embodiment of the present invention, the composition is a sunscreen composition exhibiting an UV-A protection factor of at least 10.

In connection with the above embodiments, it is to be understood that in a preferred embodiment of the present invention, the composition is a sunscreen composition exhibiting a sun protection factor (SPF) of at least 30 and an UV-A protection factor of at least 10.

Furthermore, in connection with the above embodiments, it is preferred that the composition is a sunscreen composition, wherein the UV-A protection is at least one-third of the sun protection factor. As defined in the embodiments above, if the sun protection factor of a sunscreen composition is 30, the UV-A protection of the given sunscreen composition has to be at least 10.

In another embodiment of the present invention, the composition is a sunscreen composition, wherein the composition shows a critical wavelength of at least 370 nm.

In another embodiment of the present invention, the composition is a sunscreen composition, wherein the UV-A protection is at least one-third of the sun protection factor and also shows a critical wavelength of at least 370 nm.

In this connection, it is to be understood that the compositions of the present invention are sunscreen compositions providing for efficient UV-A and UV-B protection, wherein the UV-A and UV-B protection are defined by the sun protection factor and/or the UV-A protection factor as defined above.

In another aspect, the present invention relates in one embodiment to the use of the at least one polymeric or oligomeric hydroxy phenyl triazine UV filter according to formula I as defined above for improving the photostability of a sunscreen composition comprising at least one additional UV filter, wherein the at least one further UV filter is selected from organic UV-A, UV-B, broad spectrum UV filter or a combination thereof.

In connection with the above embodiment, it is to be understood that the polymeric or oligomeric hydroxyphenyl triazine UV filter is particularly suitable for improving the photostability of the sunscreen composition. The person skilled in the art is aware that the photostability of a sunscreen composition depends on the stability of the UV filters present in the composition upon irradiation with UV light. It is common general knowledge that certain UV filters tend to isomerize upon irradiation with UV light and therefore lose their photo protective ability. Therefore, it has surprisingly been found by the inventors of the present invention that the polymeric or oligomeric hydroxy phenyl triazine UV filters according to

formula I as defined above are particularly suitable in order to improve the photostability of a sunscreen composition in combination with at least one additional UV filter, wherein the at least one further UV filter is selected from organic UV-A, UV-B, broad spectrum UV filter or a combination thereof.

In connection with the above embodiments, it is to be understood that the preferences regarding the polymeric or oligomeric hydroxy phenyl triazine UV filter and the at least one additional organic UV-A, UV-B or broad spectrum UV filter are also relevant with regard to the use for improving the photostability of a sunscreen composition as defined above.

Furthermore, in connection with the above embodiments, it is to be understood that the sunscreen composition may comprise at least one additive.

In one embodiment, the at least one additive is selected from the group consisting of emulsifier, emollients, viscosity regulators (thickeners), sensory enhancers, adjuvants, preservatives, and combinations thereof.

Preferred emulsifiers include

- glucose derivatives such as cetearyl glucoside, arachidyl glucoside, lauryl glucoside, polyglyceryl-3 methylglucose distearate, methyl glucose sesquistearate;
- sucrose derivative such as sucrose polystearate, sucrose palmitate;
- sorbitol derivatives;
- glycerides of fatty acids such as glyceryl stearate, glyceryl oleate;
- glumatic acid derivatives such as sodium stearoyl glutamate;
- sulfosuccinic acid derivatives such as disodium cetearyl sulfosuccinate;
- phosphoric acid derivatives such as potassium cetyl phosphate;
- fatty acid esters of polyglyceryl such as polyglyceryl-3-diisostearate, polyglyceryl-2-dipolyhydroxystearate;
- oxyalkenylated organomodified silicone / polysiloxane / polyalkyl / polyether copolymers and derivatives.

Preferred emollients include

- esters of linear or branched fatty acids with linear or branched fatty alcohols such as propylheptyl caprylate, coco caprylate, isopropyl myristate, ethylhexyl palmitate;
- esters of aromatic carboxylic acids with linear or branched fatty alcohols such as $C_{12}$-$C_{15}$-alkyl benzoate, ethylhexyl benzoate, phenethyl benzoate;
- dicarboxylic acid esters with linear or branched alcohols such as dibutyl adipate, dicaprylyl carbonate, diisopropyl sebacate;
- esters of hydroxycarboxylic acids with linear or branched fatty alcohols;
- esters of linear or branched fatty acids with polyhydric alcohol such as butylene glycol dicaprylate/dicaprate;
- mono-, di-, tri-glycerides based on $C_6$-$C_{18}$ fatty acids such as caprylic / capric triglycerides, coco glycerides;
- guerbet alcohols such as octyldodecynol;
- hydrocarbons such as hydrogenated polyisobutene, mineral oil, squalene, isohexadecane;
- ethers such as dicaprylyl ether;
- silicone derivatives (organomodified polysiloxanes) such as dimethylpolysiloxane, cyclic silicones.

Preferred thickeners include

- fatty alcohols such as cetyl alcohol, cetearyl alcohol, stearyl alcohol;
- fatty acids such as stearic acid;
- fatty acid esters such as myristyl stearate;
- waxes such as beeswax, carnauba wax, microcrystalline wax, ceresin, ozocerite;
- polysaccharides or derivatives such as xanthan gum, guar gum, agar gum, alginates, gellan gum, carraghenan;
- polyacrylates or homopolymers of reticulated acrylic acids or polyacrylamides such as carbomers, acrylate copolymers, acrylate / $C_{10}$-$C_{30}$-alkyl acrylate crosspolymer, acrylate / beheneth-25 methacrylate copolymer;
- silicate derivatives such as magnesium silicates;
- cellulose derivatives such as hydroxypropyl cellulose.

Preferred sensory enhancers include

- polyamide derivatives such as nylon-12;
- polymethyl methacrylates;
- silica;
- mica;

- polymethylsilsesquioxane;
- polyethylene;
- starch derivatives such as aluminum starch octenylsuccinate;
- dimethicone derivatives;
- boron nitride;
- HDI / trimethylol hexyllactone crosspolymer.

Preferred adjuvants include

- tocopherol derivatives;
- retinol derivatives;
- ascorbic acid derivatives;
- bisabolol;
- allantoin;
- panthenol;
- chelating agents (EDTA, EDDS, EGTA, phytic acid, piroctone olamine);
- ethylhexyl glycerin;
- caprylyl glycol;
- hydroxyacetophenone;
- caprylhydroxymic acid;
- propellants such as propane, butane, isobutene, dimethyl ether;
- styrene / PVP or styrene acrylamide copolymers;
- insect repellants such as butylacetylaminopropionate.

Preferred preservatives include

- phenoxyethanol;
- benzyl alcohol;
- zingerone.

Preferred perfumes are selected from the group consisting of limonene, citral, linalool, alpha-isomethylionon, geraniol, citronellol, 2-isobutyl-4-hydroxy-4-methyltetrahydropyrane, 2-tert.-pentylcyclohexylacetate, 3-methyl-5-phenyl-1-pentanol, 7-acetyl-1,1,3,4,4,6-hexamethyltetraline, adipine acid diester, alpha-amylcinnamaldehyde, alpha-methylionon, amyl C butylphenylmethylpropionalcinnamal, amylsalicylate, amylcinnamylalcohol, anisalcohol, benzoin, benzylalcohol, benzylbenzoate, benzylcinnamate, benzylsalicylate, bergamot oil, bitter orange oil, butylphenylmethylpropioal, cardamom oil, cedrol, cinnamal, cinnamylalcohol, citronnellylmethylcrotonate, lemon oil, coumarin, diethylsuccinate, ethyllinalool, eugenol, evernia furfuracea extracte, evernia prunastri extracte, farensol, guajak wood oil, hexylcinnamal, hexylsalicylate, hydroxycitronellal, lavender oil, lemon oil, linaylacetate, mandarine oil, menthyl PCA, methylheptenone, nutmeg oil, rosemary oil, sweet orange oil, terpineol, tonka bean oil, triethylcitrate, vanillin and combinations thereof.

In connection with the above preferred embodiments, it is to be understood that if the sunscreen or daily care composition comprises two or more additives, combinations of the additives as defined above are also part of the invention.

The present invention is further illustrated by the following examples.

Examples

Materials:

4-[4-(2,4-dihydroxyphenyl)-6-(4-methoxyphenyl)-1,3,5-triazin-2-yl]benzene-1,3-diol was prepared according to EP 775698 (purity > 90%).

Methyl 2-bromopropionate, CAS Number 5445-17-0, e.g. from Merck / Aldrich.

Dimer acid, hydrogenated, CAS Number: 68783-41-5: Unsaturated fatty acids, $C_{18}$, dimers, hydrogenated e.g. from Merck/ Aldrich, average Mn ≈ 570, of variable composition; (other names: 1010 Dimer, Dimer acid 1010, Empol 1010 Dimer Acid, Empol 1068, Hydrogenated C36 dimer fatty acid)

Propanoic acid, 2,2'-[[6-(4-methoxyphenyl)-1,3,5-triazine-2,4-diyl]bis[(3-hydroxy-4,1-phenylene)oxy]]bis-, dimethyl ester
CAS Registry Number 485385-10-2

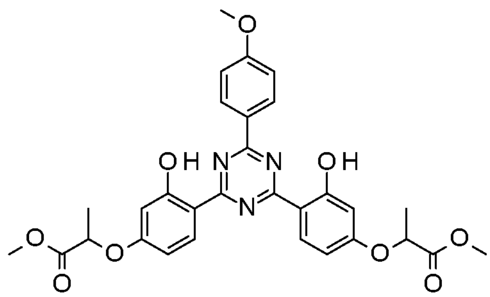

A one liter three necked flask equipped with a condenser, a magnetic stirring bar, thermometer, nitrogen and a dropping funnel was charged with 4-[4-(2,4-dihydroxyphenyl)-6-(4-methoxyphenyl)-1,3,5-triazin-2-yl]benzene-1,3-diol (111.5g, 276mmol), sodium carbonate (85.2g, 804mmol) and N,N-dimethylformamide (112.9g) and the resulting slurry was heated to 100°C. To the resulting brown mixture, a solution of methyl 2-bromo-propionate (134.16g, 803mmol) in xylene (130ml, mixture of isomers) was added during a two hours period. The reaction mixture was stirred for an additional 2 hours until TLC analysis showed no more starting material. The reaction mixture was diluted with xylene (100ml), the inorganic salts were filtered of and the filtrate was concentrated in vacuo. The residue was taken up in hot toluene (170ml) and insoluble parts were separated by filtration. The filtrate was left to cool to ambient temperature, diluted with additional toluene (100ml) and the mass was stirred until a filterable suspension resulted. The sticky solid was filtered off, washed with toluene (200ml) and dried in vacuo. Methyl-2-[3-hydroxy-4-[4-[2-hydroxy-4-(2-methoxy-1-methyl-2-oxo-ethoxy)phenyl]-6-(4-methoxyphenyl)-1,3,5-triazin-2-yl]phenoxy]propanoate (103.3g, 179mmol) was obtained as light brown solid and used without further purification.

Melting range 133-155°C (decomposition)

NMR (1H, 400MHz, CDCl3)

1.69ppm, d, 6H, 2 x OCH(*CH3*)CO; 3.82ppm, s, 6H, 2 x COOCH3; 3.90ppm, s, 3H, ArOCH3; 4.86ppm, q, 2H, 2 x O *CN*(CH3)CO; 6.41 ppm, d, 2H, aromatic H; 6.54ppm, dd, 2H, aromatic H; 6.98ppm, d, 2H, aromatic H; 8.10-8.45ppm, m, 4H, aromatic H; 13.4ppm, brs, 2H, phenolic H.

Dimer acid (hydrogenated)-, polymer with dimethyl 2,2'-[[6-(4-methoxyphenyl)-1,3,5-triazine-2,4-diyl]bis[(3-hydroxy-4,1-phenylene)oxy]]bis[propanoate] and 1,6-hexanediol, compound 13

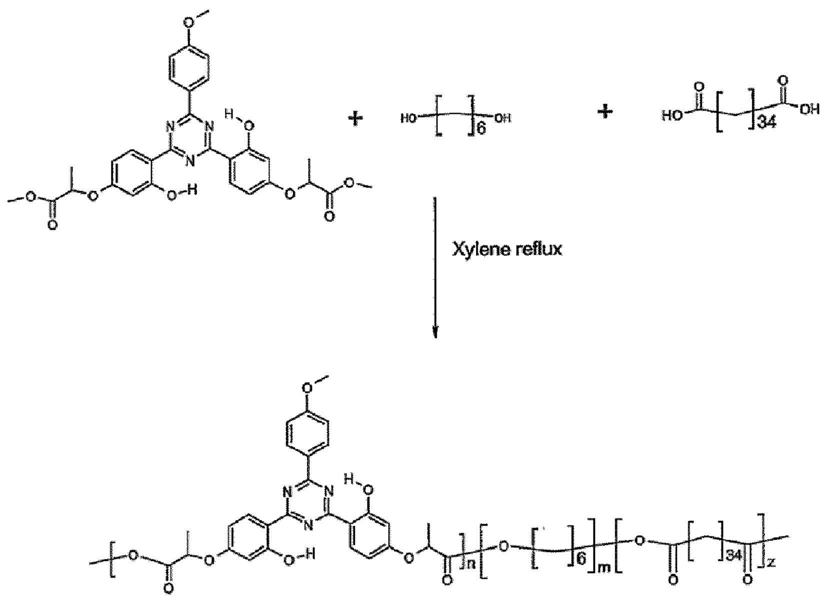


compound 13

**n:m:z=0.6:1:0.4**

A three necked flask equipped with a dean-stark condenser, thermometer, magnetic stirring bar and nitrogen inlet was charged with methyl 2-[3-hydroxy-4-[4-[2-hydroxy-4-(2-methoxy-1-methyl-2-oxo-ethoxy)phenyl]-6-(4-methoxyphenyl)-1,3,5-triazin-2-yl]phenoxy]propanoate [CAS 485385-10-2] (14.98g, 26.0mmol), dimer acid (hydrogenated) (9.90g, 17.4mmol), 1,6-hexanediol (5.19g, 43.9mmol) and xylene (100ml, mixture of isomers). p-Toluenesulfonic acid monohydrate (0.25g, 1.3mmol) was added and the reaction mixture was refluxed for nine hours. The reaction mixture was cooled to ambient temperature, diluted with 40ml of xylene and washed three times with 50 ml of water at 80°C. The organic layer was evaporated in vacuo. The residue was dissolved twice in toluene and evaporated in vacuo (120°C) to remove traces of xylene. The resulting product was used without further purification (28.08g as a light red oil).

NMR (1H, 400MHz, CDCl3):

0.73-1.00 ppm, m, terminal CH3; 1.00-1.47ppm, m, aliphatic CH2, 1.47-1.80ppm, m, aliphatic CH2, 2.16-2.63ppm, m, O-CH(*CH3*)CO; 3.49-4.30ppm, m, COOCH2 and OCH3, 6.24-7.23ppm and 8.05-8.53ppm, m, aromatic H; 13.18-13.55ppm, m, phenolic H

Solubility: > 20% in dibutyl adipate at room temperature (clear solution).

Method for SPF/UVA-protection measurements

Method for assessing in vitro Sun Protection Factor measurement (SPF)
Sunscreen composition application rate: 1.4 mg/cm$^2$ on PMMA plates (Helioplates®)
UV Transmittance analysis with Labsphere UV-1000S Transmittance Analyser

The following formula is used for calculating SPF

$$SPF = \frac{\int_{290\,nm}^{400\,nm} E_\lambda \cdot S_\lambda \cdot d\lambda}{\int_{290\,nm}^{400\,nm} E_\lambda \cdot S_\lambda \cdot T_\lambda \cdot d\lambda}$$

wherein $E_\lambda$ is erythema action spectrum, $S_\lambda$ is solar spectral irradiance and $T_\lambda$ is spectral transmittance of the sample.

Method for assessing in vitro UVA protection factor (UVA-PF)
Sunscreen composition application rate: 1.2 mg/cm$^2$ on PMMA plates (Helioplates®).

UV Transmittance analysis with Labsphere UV-1000S Transmittance Analyser.

Pre-irradiation step, to take the sun care product stability into account, via a solar simulator such as Atlas Suntest CPS+.

$$PF\ UVA = \frac{\sum_{320}^{400} \Delta\lambda}{\sum_{320}^{400} T_\lambda \cdot \Delta\lambda} = \frac{1}{T_m}$$

wherein $T_\lambda$ is the sunscreen product transmittance at a wave length $\lambda$ and $T_m$ is the mean arithmetical value of Transmittance data in the UVA range.

## Claims

1. A composition comprising at least one polymeric or oligomeric hydroxy phenyl triazine UV filter and at least one additional UV filter, wherein the at least one further UV filter is selected from organic UV-A, UV-B, broad spectrum UV filter or a combination thereof.

2. The composition according to claim 1, wherein the at least one polymeric or oligomeric hydroxy phenyl triazine UV filter is a compound according to formula (I)

$$-[A\text{-}L\text{-}D\text{-}L]_x- \quad \text{(I)}$$

in which

x is a number from 1 to 50;
A is a group of the formula (II)

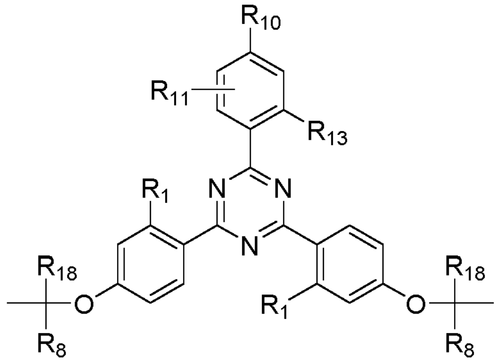

(II)

or has one of the meanings given for D, wherein formula (I) contains at least one A conforming to formula (II);

D is a divalent residue containing 2 to 60 carbon atoms comprising an aliphatic, cycloaliphatic or aromatic hydrocarbon, or said aliphatic residue substituted by OH or interrupted by O or both substituted by OH and interrupted by O; and in case that D bonds to the carbon atom of L, D also comprises methylene or a direct bond;
L stands for an ester linkage group;
the $R_1$ are independently of each other H, $OR_7$ or OH, with the proviso that at least one of $R_1$ or $R_{13}$ is OH;
the $R_7$ are independently of each other H, $C_1$-$C_{12}$-alkyl or a radical of formula (III)

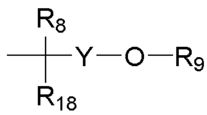

(III);

$R_8$ is H; $C_1$-$C_{18}$-alkyl, wherein the $C_1$-$C_{18}$-alkyl group is unsubstituted or substituted with one or more same or different substituents selected from the group consisting of halogen, OH, and phenyl; $C_5C_{12}$-cycloalkyl; $C_2$-$C_{18}$-alkenyl; $C_1$-$C_{18}$-alkoxy; $C_3$-$C_{18}$-alkenyloxy; $C_7$-$C_{11}$-phenylalkyl; $C_7$-$C_{11}$-alkylphenyl; $C_5$-$C_{12}$-cycloalkoxy; phenyl; or COOH;
Y is -CO-, or $C_1$-$C_{12}$-alkylene;
$R_9$ is, if Y is -CO-, $C_{20}$-$C_{60}$-alkyl unsubstituted or substituted by OH and/or interrupted by O, or is $C_{20}$-$C_{60}$-alkenyl, or is a group of formula (IV)

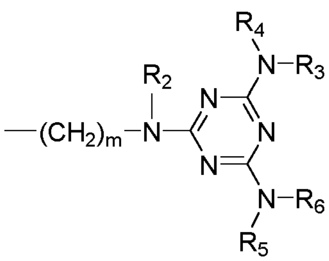

(IV)

,

wherein m is a number from 1 to 20;
or

$R_9$ is, if Y is alkylene, $C_{20}$-$C_{60}$-alkanoyl;
$R_2$, $R_3$, $R_4$, $R_5$ and $R_6$ are independently of each other H, $C_1$-$C_{38}$-alkyl which is unsubstituted or substituted by OH or $C_1$-$C_8$-alkoxy; or $C_1$-$C_{38}$-alkyl which is interrupted by an oxygen atom or a N($C_1$-$C_{18}$)alkyl group; phenyl, or $C_7$-$C_{12}$-phenylalkyl which are unsubstituted or substituted by OH or $C_1$-$C_8$-alkyl;
$R_{10}$ is H, $C_1$-$C_4$-alkyl, Cl, phenyl or a group -$OR_7$;
$R_{11}$ is H or methyl;
$R_{13}$ is H, methyl, OH or $OR_7$;
$R_{14}$ and $R_{15}$ are independently H, $C_1$-$C_8$-alkyl, Cl, or a group -$OR_7$;
$R_{18}$ is H or $C_1$-$C_8$-alkyl.

**3.** The composition according to claim 1 or 2, wherein the terminal groups of the polymer or oligomer according to formula (I) are selected from -L-D-$COOR_{12}$, -L-D-$OR_{12}$, or $OR_{12}$ if bonded to A or D; or -D-$COOR_{12}$, -D-$OR_{12}$ or -$R_{12}$ if bonded to L,
wherein
$R_{12}$ is H or $C_1$-$C_8$-alkyl.

**4.** The composition according to any one of claims 1 to 3, wherein L is an ester linkage group selected from -COO- or -OCO-.

**5.** The composition according to any one of claims 1 to 4, wherein the at least one polymeric or oligomeric hydroxy phenyl triazine UV filter is a compound according to formula (I)

$$-[A\text{-}L\text{-}D\text{-}L]_x- \qquad (I)$$

in which A is a group of formula (II), wherein

$R_{10}$ is a group $OR_7$;
$R_{11}$, $R_{13}$ is H;
$R_1$ is $OR_7$,
$R_8$, $R_{18}$ is H, or $C_1$-$C_3$-alkyl; and wherein
$R_7$ is H, or $C_1$-$C_3$-alkyl.

**6.** The composition according to any one of claims 1 to 5, wherein the at least one further organic UV-A, UV-B or broad spectrum UV filter is selected from the group consisting of micronized 5,6,5',6'-tetraphenyl-3-3'-(1,4-phenylene)bis(1,2,4-triazine) (INCI phenylene bis-diphenyltriazine), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 4-(tert.-butyl)-4'-methoxydibenzoylmethane (INCI butyl methoxydibenzoylmethane), diethylhexyl butamido triazone, 4,4',4"-(1,3,5-triazin-2,4,6-triyltriimino)tris-benzoic acid-tris(2-ethylhexyl)ester (INCI ethylhexyl triazone), bis(butylbenzoate) diaminotriazine aminopropylsiloxane, hexyl 2-[4-(diethylamino)-2-hydroxybenzoyl]benzoate (INCI diethylamino hydroxybenzoyl hexyl benzoate), 3,3,5-trimethyl-cyclohexyl salicylate (homosalate), (RS)-2-ethylhexyl-2-hydroxybenzoate (INCI ethylhexyl salicylate), 2-ethylhexyl 2-cyano-3,3-diphenyl-2-propenoate (INCI octocrylene), micronized 2,2'-methylene bis[6-(2H-benzotriazol-2-yl)-4-(1,1,3,3-tetramethylbutyl)phenol] (INCI methylene bis-benzotriazolyl tetramethylbutylphenol), 2-phenyl-1H-benzimidazole-5-sulfonic acid (INCI phenylbenzimidazole sulfonic acid), micronized 2,4,6-tris(biphenyl-4-yl)-1,3,5-triazine (INCI trisbiphenyl triazine), micronized (2-{4-[diethylamino-2-hydroxy-benzoyl)-benzoyl]-piperazine-1-carbonyl}-phenyl)-(4-diethylamino-2-hydroxy-phenyl)-methanone, 4-methoxycinnamic acid 2-ethylhexyl ester (INCI ethylhexyl methoxycinnamate), 2-(2H-benzotriazol-2-yl)-6-[(2-ethylhexyloxy)methyl]-4-methylphenol, benzylidene malonate 1, benzylidene malonate 2, benzylidene malonate 3, 2-propenoic acid 3-(4-methoxyphenyl)-2-methylphenyl ester (INCI 2-ethylhexyl methoxycinnamate), and combinations thereof.

**7.** The composition according to any one of claims 1 to 6, wherein the at least one further organic UV-A, UV-B or broad spectrum UV filter is selected from the group consisting of 4-methoxycinnamic acid 2-ethylhexyl ester (INCI ethylhexyl methoxycinnamate), hexyl 2-[4-(diethylamino)-2-hydroxybenzoyl]benzoate (INCI diethylamino hydroxybenzoyl hexyl benzoate), 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 2-phenyl-1H-benzimidazole-5-sulfonic acid (INCI phenylbenzimidazole sulfonic acid), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) and combinations thereof.

**8.** Use of at least one polymeric or oligomeric hydroxy phenyl triazine UV filter for improving the photostability of a

sunscreen composition comprising at least one additional UV filter, wherein the at least one further UV filter is selected from organic UV-A, UV-B, broad spectrum UV filter or a combination thereof.

**9.** The use according to claim 8, wherein the at least one polymeric or oligomeric hydroxy phenyl triazine UV filter is a polymeric or oligomeric hydroxy phenyl triazine UV filter according to claim 2, 3, 4, or 5.

**10.** The use according to claim 8 or 9, wherein the at least one additional UV filter is an UV filter according to claim 6 or 7.

## Patentansprüche

**1.** Zusammensetzung, umfassend mindestens einen polymeren oder oligomeren Hydroxyphenyltriazin-UV-Filter und mindestens einen zusätzlichen UV-Filter, wobei der mindestens eine weitere UV-Filter aus organischen UV-A-, UV-B-, Breitband-UV-Filtern oder einer Kombination davon ausgewählt ist.

**2.** Zusammensetzung nach Anspruch 1, wobei es sich bei dem mindestens einen polymeren oder oligomeren Hydroxyphenyltriazin-UV-Filter um eine Verbindung gemäß Formel (I)

$$-[A\text{-}L\text{-}D\text{-}L]_x- \qquad (I)$$

handelt, wobei

x für eine Zahl von 1 bis 50 steht;
A für eine Gruppe der Formel (II)

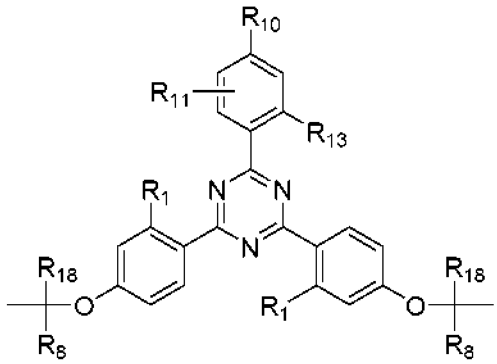

(II)

steht oder eine der für D angegebenen Bedeutungen hat, wobei Formel (I) mindestens ein A gemäß Formel (II) enthält;
D für einen zweiwertigen Molekülrest mit 2 bis 60 Kohlenstoffatomen steht, der einen aliphatischen, cycloaliphatischen oder aromatischen Kohlenwasserstoff umfasst, oder der aliphatische Rest durch OH substituiert oder durch O unterbrochen oder sowohl durch OH substituiert als auch durch O unterbrochen ist; und in dem Fall, dass D an das Kohlenstoffatom von L bindet, D auch Methylen oder eine direkte Bindung umfasst;
L für eine Ester-Verknüpfungsgruppe steht;
die Variablen $R_1$ unabhängig voneinander für H, $OR_7$ oder OH stehen, mit der Maßgabe, dass mindestens eines von $R_1$ oder $R_{13}$ für OH steht;
die Variablen $R_7$ unabhängig voneinander für H, $C_1$-$C_{12}$-Alkyl oder einen Rest der Formel (III)

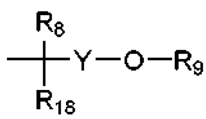

(III)

stehen,
$R_8$ für H; $C_1$-$C_{18}$-Alkyl, wobei die $C_1$-$C_{18}$-Alkylgruppe unsubstituiert oder mit einem oder mehreren gleichen oder verschiedenen Substituenten, die aus der Gruppe bestehend aus Halogen, OH und Phenyl ausgewählt

sind, substituiert ist; $C_5$-$C_{12}$-Cycloalkyl; $C_2$-$C_{18}$-Alkenyl; $C_1$-$C_{18}$-Alkoxy; $C_3$-$C_{18}$-Alkenyloxy; $C_7$-$C_{11}$-Phenylalkyl; $C_7$-$C_{11}$-Alkylphenyl; $C_3$-$C_{12}$-Cycloalkoxy; Phenyl; oder COOH steht;
Y für -CO- oder $C_1$-$C_{12}$-Alkylen steht;
$R_9$ dann, wenn Y für -CO- steht, für $C_{20}$-$C_{60}$-Alkyl, das unsubstituiert oder durch OH substituiert und/oder durch O unterbrochen ist, steht oder für $C_{20}$-$C_{60}$-Alkenyl steht oder für eine Gruppe der Formel (IV)

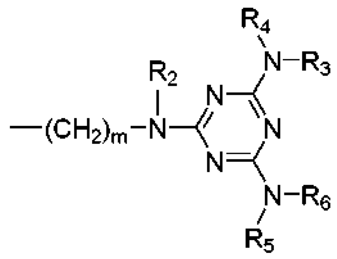

(IV)

steht,
wobei m für eine ganze Zahl von 1 bis 20 steht; oder
$R_9$ dann, wenn Y für Alkylen steht, für $C_{20}$-$C_{60}$-Alkanoyl steht;
$R_2$, $R_3$, $R_4$, $R_5$ und $R_6$ unabhängig voneinander für H, $C_1$-$C_{38}$-Alkyl, das unsubstituiert oder durch OH oder $C_1$-$C_8$-Alkoxy substituiert ist; oder
$C_1$-$C_{38}$-Alkyl, das durch ein Sauerstoffatom oder eine $N(C_1$-$C_{18})$Alkylgruppe unterbrochen ist;
Phenyl oder $C_7$-$C_{12}$-Phenylalkyl, die unsubstituiert oder durch OH oder $C_1$-$C_8$-Alkyl substituiert sind; stehen;
$R_{10}$ für H, $C_1$-$C_4$-Alkyl, Cl, Phenyl oder eine Gruppe -$OR_7$ steht;
$R_{11}$ für H oder Methyl steht;
$R_{13}$ für H, Methyl, OH oder $OR_7$ steht;
$R_{14}$ und $R_{15}$ unabhängig für H, $C_1$-$C_8$-Alkyl, Cl oder eine Gruppe -$OR_7$ stehen;
$R_{18}$ für H oder $C_1$-$C_8$-Alkyl steht.

**3.** Zusammensetzung nach Anspruch 1 oder 2, wobei die Endgruppen des Polymers oder Oligomers gemäß Formel (I) aus -L-D-$COOR_{12}$, -L-D-$OR_{12}$ oder $OR_{12}$, wenn sie an A oder D gebunden sind; oder -D-$COOR_{12}$, -D-$OR_{12}$ oder -$R_{12}$, wenn sie an L gebunden sind, ausgewählt sind,
wobei
$R_{12}$ für H oder $C_1$-$C_8$-Alkyl steht.

**4.** Zusammensetzung nach einem der Ansprüche 1 bis 3, wobei L für eine Ester-Verknüpfungsgruppe steht, die aus -COO- oder -OCO- ausgewählt ist.

**5.** Zusammensetzung nach einem der Ansprüche 1 bis 4, wobei es sich bei dem mindestens einen polymeren oder oligomeren Hydroxyphenyltriazin-UV-Filter um eine Verbindung gemäß Formel (I)

$$-[A\text{-}L\text{-}D\text{-}L]_x- \qquad (I)$$

handelt, wobei A für eine Gruppe der Formel (II) steht,
wobei
$R_{10}$ für eine Gruppe $OR_7$ steht;
$R_{11}$ und $R_{13}$ für H stehen;
$R_1$ für $OR_7$ steht;
$R_8$ und $R_{18}$ für H oder $C_1$-$C_3$-Alkyl stehen; und wobei
$R_7$ für H oder $C_1$-$C_3$-Alkyl steht.

**6.** Zusammensetzung nach einem der Ansprüche 1 bis 5, wobei der mindestens eine weitere organische UV-A-, UV-B- oder Breitband-UV-Filter aus der Gruppe bestehend aus mikronisiertem 5,6,5',6'-Tetraphenyl-3-3'-(1,4-phenylen)bis(1,2,4-triazin) (INCI Phenylene Bis-Diphenyltriazine), 2,4-Bis{[4-(2-ethylhexyloxy)-2-hydroxy]phenyl}-6-(4-methoxyphenyl)-1,3,5-triazin (INCI Bis-Ethylhexyloxyphenol Methoxyphenyl Triazine), 4-(tert.-Butyl)-4'-methoxydibenzoylmethan (INCI Butyl Methoxydibenzoylmethane), Diethylhexylbutamidotriazon, 4,4',4"-(1,3,5-Triazin-2,4,6-triyltriimino)trisbenzoesäuretris(2-ethylhexyl)ester (INCI Ethylhexyl Triazone), Bis(butylbenzoat)diaminotriazinaminopropylsiloxan, Hexyl-2-[4-(diethylamino)-2-hydroxybenzoyl]benzoat (INCI Diethylamino Hydroxybenzoyl Hexyl

Benzoate), 3,3,5-Trimethylcyclohexylsalicylat (Homosalate), (RS)-2-Ethylhexyl-2-hydroxybenzoat (INCI Ethylhexyl Salicylate), 2-Ethylhexyl-2-cyano-3,3-diphenyl-2-propenoat (INCI Octocrylene), mikronisiertem 2,2’-Methylen-bis[6-(2H-benzotriazol-2-yl)-4-(1,1,3,3-tetramethylbutyl)phenol] (INCI Methylene Bis-Benzotriazolyl Tetramethylbutylphenol), 2-Phenyl-1H-benzimidazol-5-sulfonsäure (INCI Phenylbenzimidazole Sulfonic Acid), mikronisiertem 2,4,6-Tris(biphenyl-4-yl)-1,3,5-triazin (INCI Trisbiphenyl Triazine), mikronisiertem (2-{4-[Diethylamino-2-hydroxybenzoyl)benzoyl]piperazin-1-carbonyl}phenyl)-(4-diethylamino-2-hydroxyphenyl)methanon, 4-Methoxyzimtsäure-2-ethylhexylester (INCI Ethylhexyl Methoxycinnamate), 2-(2H-Benzotriazol-2-yl)-6-[(2-ethylhexyloxy)methyl]-4-methylphenol, Benzylidenmalonat 1, Benzylidenmalonat 2, Benzylidenmalonat 3, 2-Propensäure-3-(4-methoxyphenyl)-2-methylphenylester (INCI 2-Ethylhexyl Methoxycinnamate) und Kombinationen davon ausgewählt ist.

**7.** Zusammensetzung nach einem der Ansprüche 1 bis 6, wobei der mindestens eine weitere organische UV-A-, UV-B- oder Breitband-UV-Filter aus der Gruppe bestehend aus 4-Methoxyzimtsäure-2-ethylhexylester (INCI Ethylhexyl Methoxycinnamate), Hexyl-2-[4-(diethylamino)-2-hydroxybenzoyl]benzoat (INCI Diethylamino Hydroxybenzoyl Hexyl Benzoate), 4,4’,4"-(1,3,5-Triazin-2,4,6-triyltriimino)trisbenzoesäuretris(2-ethylhexyl)ester (INCI Ethylhexyl Triazone), (RS)-2-Ethylhexyl-2-hydroxybenzoat (INCI Ethylhexyl Salicylate), 2-Phenyl-1H-benzimidazol-5-sulfonsäure (INCI Phenylbenzimidazole Sulfonic Acid), 2,4-Bis{[4-(2-ethylhexyloxy)-2-hydroxy]phenyl}-6-(4-methoxyphenyl)-1,3,5-triazin (INCI Bis-Ethylhexyloxyphenol Methoxyphenyl Triazine) und Kombinationen davon ausgewählt ist.

**8.** Verwendung von mindestens einem polymeren oder oligomeren Hydroxyphenyltriazin-UV-Filter zur Verbesserung der Lichtstabilität einer Sonnenschutzzusammensetzung, die mindestens einen zusätzlichen UV-Filter umfasst, wobei der mindestens eine weitere UV-Filter aus organischen UV-A-, UV-B-, Breitband-UV-Filtern oder einer Kombination davon ausgewählt ist.

**9.** Verwendung nach Anspruch 8, wobei es sich bei dem mindestens einen polymeren oder oligomeren Hydroxyphenyltriazin-UV-Filter um einen polymeren oder oligomeren Hydroxyphenyltriazin-UV-Filter nach Anspruch 2, 3, 4 oder 5 handelt.

**10.** Verwendung nach Anspruch 8 oder 9, wobei es sich bei dem mindestens einen zusätzlichen UV-Filter um einen UV-Filter gemäß Anspruch 6 oder 7 handelt.

## Revendications

**1.** Composition comprenant au moins un filtre UV de type hydroxyphényltriazine polymérique ou oligomérique et au moins un filtre UV additionnel, dans laquelle l’au moins un filtre UV supplémentaire est choisi parmi un filtre organique du type UV-A, UV-B ou UV à large spectre ou une combinaison correspondante.

**2.** Composition selon la revendication 1, dans laquelle l’au moins un filtre UV de type hydroxyphényltriazine polymérique ou oligomérique est un composé selon la formule (I)

$$-[A\text{-}L\text{-}D\text{-}L]_x- \qquad (I)$$

dans laquelle

x est un nombre de 1 à 50 ;
A est un groupe de la formule (II)

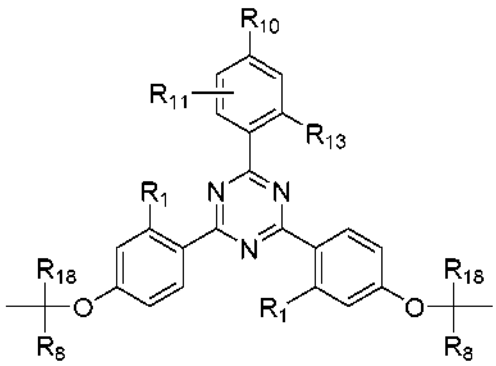

(II)

ou possède l'une des significations données pour D, dans laquelle la formule (I) contient au moins un A conforme à la formule (II) ;

D est un radical divalent contenant 2 à 60 atomes de carbone comprenant un hydrocarbure aliphatique, cycloaliphatique ou aromatique, ou ledit radical aliphatique étant substitué par OH ou interrompu par O ou à la fois substitués par OH et interrompus par O ; et dans le cas où D est lié à l'atome de carbone de L, D comprend également méthylène ou une liaison directe ;

L représente un groupe de liaison ester ;

les $R_1$ sont indépendamment les uns des autres H, $OR_7$ ou OH, à condition qu'au moins l'un parmi $R_1$ ou $R_{13}$ soit OH ; les $R_7$ sont indépendamment les uns des autres H, $C_1$-$C_{12}$-alkyle ou un radical de formule (III)

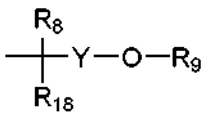

(III) ;

$R_8$ est H ; $C_1$-$C_{18}$-alkyle, dans laquelle le groupe $C_1$-$C_{18}$-alkyle est non substitué ou substitué par un ou plusieurs substituants identiques ou différents choisis dans le groupe constitué par halogène, OH, et phényle ; $C_5$-$C_{12}$-cycloalkyle ; $C_2$-$C_{18}$-alcényle ; $C_1$-$C_{18}$-alcoxy ; $C_3$-$C_{18}$-alcényloxy ; $C_7$-$C_{11}$-phénylalkyle ; $C_7$-$C_{11}$-alkylphényle ; $C_5$-$C_{12}$-cycloalcoxy ; phényle ; ou COOH ;

Y est -CO-, ou $C_1$-$C_{12}$-alkylène ;

$R_9$ est, si Y est -CO-, $C_{20}$-$C_{60}$-alkyle non substitué ou substitué par OH et/ou interrompu par O, ou est $C_{20}$-$C_{60}$-alcényle, ou est un groupe de formule (IV)

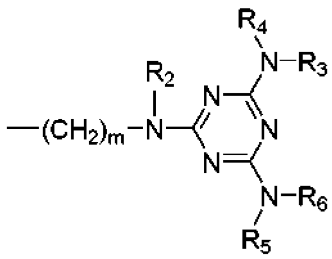

(IV),

dans laquelle m est un nombre de 1 à 20 ;

ou

$R_9$ est, si Y est alkylène, $C_{20}$-$C_{60}$-alcanoyle ;

$R_2$, $R_3$, $R_4$, $R_5$ et $R_6$ sont indépendamment les uns des autres H, $C_1$-$C_{38}$-alkyle qui est non substitué ou substitué par OH ou $C_1$-$C_8$-alcoxy ; ou

$C_1$-$C_{38}$-alkyle qui est interrompu par un atome d'oxygène ou un groupe N($C_1$-$C_{18}$) alkyle ;

phényle, ou $C_7$-$C_{12}$-phénylalkyle qui sont non substitués ou substitués par OH ou $C_1$-$C_8$-alkyle ;

$R_{10}$ est H, $C_1$-$C_4$-alkyle, Cl, phényle ou un groupe $-OR_7$ ;

$R_{11}$ est H ou méthyle ;

$R_{13}$ est H, méthyle, OH ou $OR_7$ ;

$R_{14}$ et $R_{15}$ sont indépendamment H, $C_1$-$C_8$-alkyle, Cl, ou un groupe $-OR_7$ ;

$R_{18}$ est H ou $C_1$-$C_8$-alkyle.

**3.** Composition selon la revendication 1 ou 2, dans laquelle les groupes terminaux du polymère ou de l'oligomère selon la formule (I) sont choisis parmi -L-D-$COOR_{12}$, -L-D-$OR_{12}$, ou $OR_{12}$ si lié à A ou D ; ou -D-$COOR_{12}$, -D-$OR_{12}$ ou -$R_{12}$ si lié à L,
dans laquelle
$R_{12}$ est H ou $C_1$-$C_8$-alkyle.

**4.** Composition selon l'une quelconque des revendications 1 à 3, dans laquelle L est un groupe de liaison ester choisi parmi -COO- ou -OCO-.

**5.** Composition selon l'une quelconque des revendications 1 à 4, dans laquelle l'au moins un filtre UV de type hydroxyphényltriazine polymérique ou oligomérique est un composé selon la formule (I)

$$-[A\text{-}L\text{-}D\text{-}L]_x- \qquad (I)$$

dans laquelle A est un groupe de formule (II), dans laquelle
$R_{10}$ est un groupe $OR_7$ ;
$R_{11}$, $R_{13}$ est H ;
$R_1$ est $OR_7$,
$R_8$, $R_{18}$ est H, ou $C_1$-$C_3$-alkyle ; et dans laquelle
$R_7$ est H, ou $C_1$-$C_3$-alkyle.

**6.** Composition selon l'une quelconque des revendications 1 à 5, dans laquelle l'au moins un filtre organique du type UV-A, UV-B ou UV à large spectre supplémentaire est choisi dans le groupe constitué par 5,6,5',6'-tétraphényl-3-3'-(1,4-phénylène)bis(1,2,4-triazine) micronisée (INCI phenylene bis-diphenyltriazine), 2,4-bis-{[4-(2-éthyl-hexyloxy)-2-hydroxy]-phényl}-6-(4-méthoxyphényl)-1,3,5 triazine (INCI bis-ethylhexyloxyphenol methoxyphenyl triazine), 4-(tert.-butyl)-4'-méthoxydibenzoylméthane (INCI butyl methoxydibenzoylmethane), diéthylhexyl butamido triazone, acide 4,4',4"-(1,3,5-triazin-2,4,6-triyltriimino)tris-benzoïque tris(2-éthylhexyl)ester (INCI ethylhexyl triazone), bis(butylbenzoate) diaminotriazine aminopropylsiloxane, 2-[4-(diéthylamino)-2-hydroxybenzoyl]benzoate d'hexyle (INCI diethylamino hydroxybenzoyl hexyl benzoate), salicylate de 3,3,5-triméthyl-cyclohexyle (homosalate), (RS)-2-hydroxybenzoate de 2-éthylhexyle (INCI ethylhexyl salicylate), 2-cyano-3,3-diphényl-2-propénoate de 2-éthylhexyle (INCI octocrylène), 2,2'-méthylène bis[6-(2H-benzotriazol-2-yl)-4-(1,1,3,3-tétraméthylbutyl)phénol] micronisé (INCI methylene bis-benzotriazolyl tetramethylbutylphenol), acide 2-phényl-1H-benzimidazole-5-sulfonique (INCI phenylbenzimidazole sulfonic acid), 2,4,6-tris(biphényl-4-yl)-1,3,5-triazine (INCI trisbiphsnyl triazine) micronisé, (2-{4-[diéthylamino-2-hydroxy-benzoyl)-benzoyl]-pipérazine-1-carbonyl}-phényl)-(4-diéthylamino-2-hydroxy-phényl)-méthanone micronisée, ester de 2-éthylhexyle d'acide 4-méthoxycinnamique (INCI methoxycinnamate d'ethylhexyle), 2-(2H-benzotriazol-2-yl)-6-[(2-éthylhexyloxy)méthyl]-4-méthylphénol, malonate de benzylidène 1, malonate de benzylidène 2, malonate de benzylidène 3, ester 3-(4-méthoxyphényl)-2-méthylphényle d'acide 2-propénoïque (INCI methoxycinnamate de 2-ethylhexyle), et des combinaisons correspondantes.

**7.** Composition selon l'une quelconque des revendications 1 à 6, dans laquelle l'au moins un filtre organique du type UV-A, UV-B ou UV à large spectre supplémentaire est choisi dans le groupe constitué par ester 2-éthylhexylique de l'acide 4-méthoxycinnamique (INCI methoxycinnamate d'ethylhexyle), 2-[4-(diéthylamino)-2-hydroxybenzoyl]benzoate d'hexyle (INCI diethylamino hydroxybenzoyl hexyl benzoate), acide 4,4',4"-(1,3,5-triazin-2,4,6-triyltriimino)tris-benzoïque ester de tris(2-éthylhexyle) (INCI éthylhexyl triazone), (RS)-2-hydroxybenzoate de 2-éthylhexyle (INCI salicylate d'ethylhexyle), acide 2-phényl-1H-benzimidazole-5-sulfonique (INCI phenylbenzimidazole sulfonic acid), 2,4-bis-{[4-(2-éthyl-hexyloxy)-2-hydroxy]-phényl}-6-(4-méthoxyphényl)-1,3,5 triazine (INCI bis-ethylhexyloxyphenol methoxyphenyl triazine) et des combinaisons correspondantes.

**8.** Utilisation d'au moins un filtre UV de type hydroxyphényltriazine polymérique ou oligomérique pour l'amélioration de la photostabilité d'une composition d'écran solaire comprenant au moins un filtre UV additionnel, dans laquelle l'au moins un filtre UV supplémentaire est choisi parmi un filtre organique du type UV-A, UV-B ou UV à large spectre ou une combinaison correspondante.

**9.** Utilisation selon la revendication 8, dans laquelle l'au moins un filtre UV de type hydroxyphényltriazine polymérique ou oligomérique est un filtre UV de type hydroxyphényltriazine polymérique ou oligomérique selon la revendication 2, 3, 4 ou 5.

**10.** Utilisation selon la revendication 8 ou 9, dans laquelle l'au moins un filtre UV additionnel est un filtre UV selon la revendication 6 ou 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014004176 A1
- WO 03004557 A1
- EP 434608 A
- US 3118887 A
- EP 165608 A
- US 4826978 A
- US 5736597 A
- US 5986233 A
- US 5959008 A
- EP 775698 A


### Non-patent literature cited in the description


- **H. BRUNETTI ; C.E. LÜTHI.** *Helv. Chim. Acta,* 1972, vol. 55, 1566
- *IP.com Journal,* 2009, vol. 9 (1B), 17
- *CHEMICAL ABSTRACTS,* 5445-17-0
- *CHEMICAL ABSTRACTS,* 68783-41-5
- *CHEMICAL ABSTRACTS,* 485385-10-2